(19) Europäisches Patentamt
European Patent Office
Office européen des brevets

(11) EP 1 404 376 B1

(12) **EUROPEAN PATENT SPECIFICATION**

(45) Date of publication and mention of the grant of the patent:
**14.10.2009 Bulletin 2009/42**

(21) Application number: **01987641.6**

(22) Date of filing: **19.10.2001**

(51) Int Cl.:
***A61K 51/00*** *(2006.01)*

(86) International application number:
**PCT/US2001/050802**

(87) International publication number:
**WO 2002/032294 (25.04.2002 Gazette 2002/17)**

# (54) RADIOPHARMACEUTICAL FORMULATIONS

RADIOPHARMAZEUTISCHE FORMULIERUNGEN

FORMULATIONS DE PRODUITS RADIOPHARMACEUTIQUES

(84) Designated Contracting States:
**AT BE CH CY DE DK ES FI FR GB GR IE IT LI LU MC NL PT SE TR**

(30) Priority: **19.10.2000 US 241782 P**

(43) Date of publication of application:
**07.04.2004 Bulletin 2004/15**

(73) Proprietor: **Bracco Imaging SPA**
**20134 Milan (IT)**

(72) Inventors:
- **CAGNOLINI, Aldo**
  **Edison, NJ 08817 (US)**
- **LINDER, Karen, E.**
  **Kingston, NJ 08528 (US)**
- **MARINELLI, Edmund, R.**
  **Lawrenceville, NJ 08648 (US)**

(74) Representative: **Bösl, Raphael Konrad et al**
**Isenbruck Bösl Hörschler Wichmann Huhn LLP**
**Prinzregentenstrasse 68**
**81675 München (DE)**

(56) References cited:
**WO-A-02/092005**
**GB-A- 2 231 267**
**US-A- 4 229 427**
**US-A- 5 262 175**
**US-A- 5 318 993**
**US-A- 5 665 329**
**US-A- 5 741 912**

- **KULKARNI M G ET AL: "1,4 - BENZOQUINONE : A NEW SELECTIVE REAGENT FOR OXIDATION OF ALCOHOLS" TETRAHEDRON LETTERS, ELSEVIER, AMSTERDAM, NL, vol. 31, no. 31, 23 July 1990 (1990-07-23), pages 4497-4500, XP000136383 ISSN: 0040-4039**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 1 404 376 B1

Printed by Jouve, 75001 PARIS (FR)

## Description

### Field of the Invention

The present invention relates to a kit for preparing a metal radionuclide-containing radiopharmaceutical, the kit comprising a predetermined quantity of a complexing ligand and a predetermined quantity of an oxidant, a pharmaceutical composition comprising a metal radionuclide-containing radiopharmaceutical and an oxidant, and a method for making a metal radionuclide-containing radiopharmaceutical composition.

### Background of the Invention

Because of the short half-life of the radionuclide, radiopharmaceuticals are generally prepared within hours of their administration, using formulations for the preparation of the product that contain all reagents except for the radionuclide.

Radiopharmaceuticals based, for example, on the radionuclides $^{99m}Tc$, $^{51}Cr$, $^{67}Cu$, $^{97}Ru$, $^{188}Re$, $^{186}Re$, and $^{199}Au$, can be prepared by reaction with a reducing agent, such as a stannous source, to reduce the metal in an oxidized state to a reduced state that can coordinate with the desired ligand. Useful reducing agents include, for example, stannous chloride, stannous pyrophosphate, stannous fluoride, stannous tartrate, stannous glucoheptonate, stannous DTPA, sodium or other salts of borohydride, sodium dithionite, Cu(I) salts, formamidine sulphinic acid and the like.

Radiopharmaceuticals are usually prepared from a freeze-dried formulated product in "kit" form. These kits typically contain all of the reagents required for the preparation of the radioactive product except the radionuclide and diluents such as saline. For example, the kit contains a complexing ligand able to coordinate to a radionuclide, such as technetium, and reducing agent, such as a stannous source, that can reduce the radionuclide to a reduced state that can coordinate to the ligating molecule (for example, technetium is reduced from $^{99m}TcO_4^-$. the form of technetium that is widely available, to a state that can coordinate with the desired ligand).

Examples of complexing ligands are those found in the following $^{99m}Tc$ kit radiopharmaceuticals: Kits for the preparation of $^{99m}Tc$-bis(bis(2-ethyloxyethyl)phosphino)ethane ($^{99m}Tc$-Myoview, $^{99m}Tc$-Tetrofosmin). $^{99m}Tc$-trans(1,2-bis(dehydro-2,2,5,5,-tetramethyl-3-furanone-4-methylene-amino)ethane)-tris(3-methoxy-1-propyl)phosphine ($^{99m}Tc$-Technescan Q12, $^{99m}Tc$-Furifosmin), $^{99m}Tc$-melhoxylsobutylisonitrile ($^{99}Tc$-MIB1), $^{99}Tc$-1,1-ethylenecysteine diethylester dimer ($^{99m}Tc$-ECD), $^{99m}Tc$-d,1-hexamethylene propyleneamine oxime ($^{99m}Tc$-HMPAO), $^{99m}Tc$-diethylenetriaminepentaacetic acid ($^{99m}Tc$-DTPA), $^{99m}Tc$-ethylene hepatobiliary iminodiacetic acid ($^{99m}Tc$-EHIDA), $^{99m}Tc$-1,1-ethylenecysteine dimer ($^{99m}Tc$-EC), $^{99m}Tc$-mercaptoacetylglycylglycylglycine ($^{99m}Tc$-MAG3), $^{99m}Tc$-dimercaptosuccinic acid ($^{99m}Tc$-DMSA), and $^{99m}Tc$-methylene diphosphonate ($^{99m}Tc$-MDP).

Alternatively, radiopharmaceuticals may be formed by reacting an appropriate complexing agent with a radionuclide, such as technetium or rhenium, that has been prereduced in the presence of a weakly coordinating labile ligand known as a "transfer" or "exchange" ligand. This process is known as ligand exchange and is well known to those skilled in the art.

The useful life of a reconstituted radiopharmaceutical product is dictated by the radiochemical purity (RCP) of this product (i.e. the percentage of the radiolabeled product that is the compound of interest). A common problem in this area is that the desired radiolabeled compound often begins to decompose immediately after the product is made. If this rate of decomposition is fast, an unacceptable level of degradation is reached before the radiolabeled product can be administered to the patient to provide beneficial effect. In general, after the RCP drops below 90%, the compound can no longer be used. Additives that can increase the post-reconstitution shelf-life of the product are therefore important. This is especially important when the radiolabeling is performed at a central radiolabeling facility, and the radiolabeled compound is then shipped to the site of patient administration.

WO 02/092005 describes methods for stabilizing radionuclide-containing compositions against degradation caused by free radicals generated by radionuclides. In this context, the document discloses the use of iodide ions as scavengers to the generated free radicals and thus, preventing or lessenening of the respective degradation of radionuclide-containing compositions, preferably comprising technetium-99m.

GB-A-2 231 267 discloses the method of stabilizing a radiopharmaceutical complex composition formed by reaction of a radiopharmaceutical element selected from technetium, rhenium, manganese, lanthanum, a transitional element, or a Rare Earth element, preferably technetium-99m as radiopharmaceutical element, and an organic complexing compound capable of complexing said radiopharmaceutical element, preferably the hexamethyl derivative of propylene-amineoxime as complexing compound, by addition of a pharmaceutically acceptable weak oxidizing agent to the reaction mixture, preferably sodium hypochlorite.

Likewise, US 5,262,175 discloses a method of stabilizing a radiopharmaceutical composition which is preferably comprised of Technetium-99m as radiopharmaceutical element and the hexamethyl derivative of propyleneamineoxime as complexing compound by adding a sufficient amount of a pharmacologically acceptable weak oxidizing agent such

as sodium hypochlorite.

M. G. Kulkarni et al. (Tetrahedron Letters, 30, 31, 4497-4500, 1990) discloses the use of 1,4-benzoquinone as a highly selective reagent for the oxidation of aryl conjugated primary allylic alcohols in the presence of benzylic and aryl conjugated secondary allylic alcohols.

Typically, the stabilizers that are added are antioxidants. The purpose of these stabilizers is to prevent any stannous ion [Sn(2+)] that remains in the vial after the formation of the desired complex from being oxidized to Sn(4+) and to keep the complex of interest from being oxidized back to the starting material ($TcO_4^-$). The use of antioxidants, such as gentisic acid and ascorbic acid, as stabilizers for diagnostic radiopharmaceuticals based on $^{99m}Tc$ has been described in various references, including: EP-A-46067; Tofe ct al, Radiopharm. 2, Proc. Int. Symp., 2nd (1979); Solanki et al, C. B. Nucl. Med. Commun. (1994), 15(9). 718-22; Cleyhens et al, Lab. Nucl. Med. Radiophann., Nuklearmedizin, Suppl. (1991), 27 133-5; EP-A-313712; DE 3323375; Res. Discl. (1989), 305 666. In general, the goal of the use of these stabilizers is to prevent oxidation of the product and maintain stannous levels for as long as possible.

However, in some cases, excess antioxidant or reducing agent in the vial may have a deleterious effect on the long-term post reconstitution stability of radiopharmaceutical products. For example, compounds which contain reducible functional groups such as aromatic nitro groups, nitroimidazoles, and disulfide bonds that can react with the stannous ion may be reduced to forms that are no longer biologically active (in molecules that target biological targets), or the central metal can be reduced to lower oxidation states that do not have the same desirable characteristics as the desired product Previous attempts to stabilize radiopharmaceutical complexes have used oxidizing agents, but have failed to provide a radiopharmaceutical with greater than 90% radiochemical purity after even thirty minutes. In addition, the oxidizing agents used in these earlier attempts cannot be added prior to complex formation, and/or cannot be lyophilized for use in kits.

Thus, there is a need for stabilizers that can inhibit degradation of a diagnostic or radiotherapeutic radiopharmaceutical by further reduction of either the ligand molecule that is coordinated to the radioactive metal or of the central metal itself. In particular, there is a need for radiopharmaceuticals with radiochemical purity of greater than 90% for up to six hours after reconstitution. There is also a need in the art for radiopharmaceutical preparations with fewer impurities. Moreover, there is a need for radiopharmaceutical preparations containing an oxidizing agent that may be lyophilized for use in kits. In addition, there is a need for radiopharmaceutical preparations which allow addition of an oxidizing agent prior to complex formation. Such preparations would permit inclusion of the oxidizing agent in the same vial as the complexing ligand, thereby decreasing manufacturing cost and increasing ease of use.

Summary of the Invention

The present invention provides a kit for preparing a metal radionuclide-containing radiopharmaceutical, the kit comprising a predetermined quantity of a complexing ligand and a predetermined quantity of an oxidant, wherein the oxidant has the following structure:

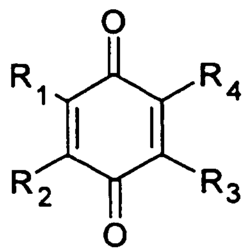

or

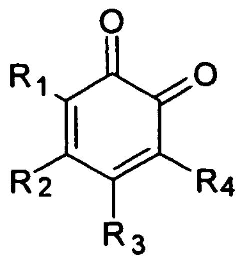

where $R_1$, $R_2$, $R_3$, and $R_4$ are independently selected from hydrogen, hydroxyl, halogen, methyl, alkoxy, aryl, heteroaryl, $NO_2$ and

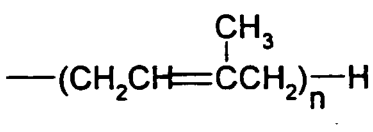

where n is an integer from 0 to 12; or where $R_1$ and $R_2$ taken together form a ring and $R_3$ and $R_4$ are as defined above,

and the radiopharmaceutical has a radiochemical purity of greater than 90% at six hours after reconstitution, in particular wherein the radiochemical purity is greater than 92 % at six hours after reconstitution, in particular, wherein the radiochemical purity is greater than 95 % at six hours after reconstitution

The present invention can be applied to a wide variety of radiopharmaceuticals that are formed by reduction of a radioactive metal using an excess of reducing agent in < the presence of a chelating or a complexing ligand (such as monodentate and polydentate ligands known in the art), and wherein further reduction of either

a. the central radioactive metal ion, or
b. a reducible moiety on the chelating or complexing ligand can take place after the formation of the complex. The oxidants of the present invention can be used to prevent such further reduction.

In the first case, reaction with excess reductant after the desired compound has formed may cause the central metal to be reduced from the desired oxidation state (e.g. Tc or Re(V)) to a lower one (e.g. Tc or Re (IV or III)).

In the second case, excess reductant present after the desired compound has formed may cause a readily reducible group on the chelating or complexing ligand to be further reduced.

The present invention also provides a pharmaceutical composition comprising a metal radionuclide-containing radiopharmaceutical and an oxidant, wherein the oxidant has the following structure:

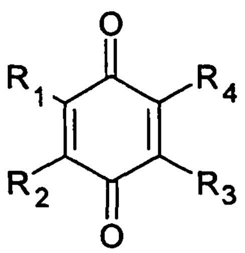

or

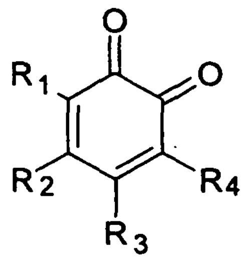

where $R_1$, $R_2$, $R_3$, and $R_4$ are independently selected from hydrogen, hydroxyl, halogen, methyl, alkoxy, aryl, heteroaryl, $NO_2$ and

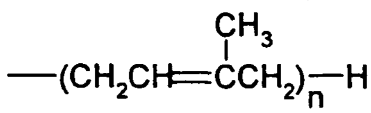

where n is an integer from 0 to 12; or where $R_1$ and $R_2$ taken together form a ring and $R_3$ and $R_4$ are as defined above, and the radiopharmaceutical has a radiochemical purity of greater than 90% at six hours after reconstitution. Preferably, the radiopharmaceutical has a radiochemical purity of greater than 92% at six hours after reconstitution, in particular, wherein the composition has a radiochemical purity of greater than 94% at six hours after reconstitution, in particular, wherein the composition has a radiochemical purity of greater than 96% at six hours after reconstitution. In some embodiments of the invention, the oxidant is a quinone, such as benzoquinone. In preferred embodiments, the oxidant is an ubiquinone, most preferably coenzyme $Q_0$.

The present invention also provides a kit for the preparation of a metal radionuclide-containing radiopharmaceutical, the kit comprising a lyophilized composition comprising a predetermined quantity of a complexing ligand and a predetermined quantity of an oxidant, wherein the oxidant has the following structure:

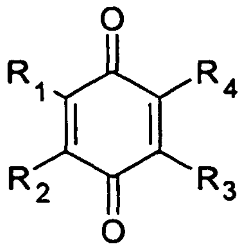

or

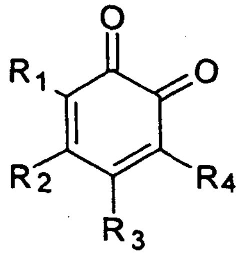

where $R_1$, $R_2$, $R_3$, and $R_4$ are independently selected from hydrogen, hydroxyl, halogen, methyl, alkoxy, aryl, heteroaryl, $NO_2$ and

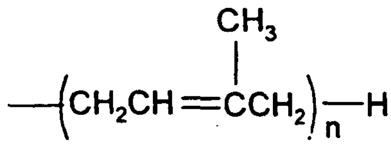

where n is an integer from 0 to 12; or where $R_1$ and $R_2$ taken together form a ring and $R_3$ and $R_4$ are as defined above. In a preferred embodiment, the kit includes a vial containing both an oxidant and a complexing ligand. In another preferred embodiment, the kit includes a lyophilized composition, which includes a predetermined amount of a complexing ligand and predetermined amount of an oxidant. Radiopharmaceuticals prepared from kits of the invention have a radiochemical purity of greater than 90% at six hours after reconstitution, more preferably, greater than 92% at six hours after reconstitution, and most preferably, greater than 95% at six hours after reconstitution. In other embodiments, the kits of the invention include a reducing agent. In some embodiments of the invention, the oxidant included in the kit is a quinone, such as benzoquinone. In preferred embodiments, the oxidant is an ubiquinone, most preferably coenzyme $Q_0$.

The present invention also provides a method of making a metal radionuclide-containing radiopharmaceutical composition, comprising the steps of:

a) contacting a complexing ligand with an oxidant wherein the oxidant has the following structure:

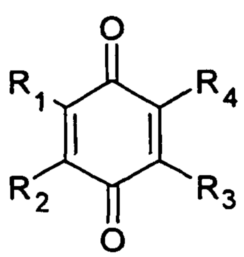

or

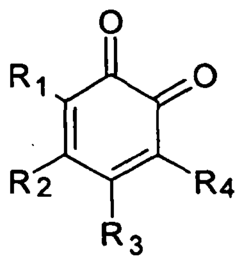

where $R_1$, $R_2$, $R_3$, and $R_4$ are independently selected from hydrogen, hydroxyl, halogen, methyl, alkoxy, aryl, heteroaryl, $NO_2$ and

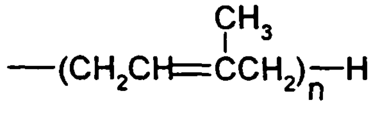

where n is an integer from 0 to 12; or where $R_1$ and $R_2$ taken together form a ring and $R_3$ and $R_4$ are as defined above;
b) followed by contacting the complexing ligand with the radionuclide to form a complex, wherein the radiopharmaceutical composition has a radiochemical purity of greater than 90% at six hours after reconstitution.

In a specific embodiment the radiopharmaceutical composition has a radiochemical purity of greater than 92% approximately six hours after the complex is formed, in particular, wherein the radiopharmaceutical composition has a radiochemical purity of greater than 94% approximately six hours after the complex is formed, in particular, wherein the radiopharmaceutical composition has a radiochemical purity of greater than 96% approximately six hours after the complex is formed. In some embodiments of the invention, the oxidant used in the method is a quinone, such as benzoquinone. In preferred embodiments, the oxidant used in the method is an ubiquinone, most preferably coenzyme $Q_0$.

Among those benefits and improvements that have been disclosed, other objects and advantages of this invention will become apparent from the following description. The tables and charts constitute a part of this specification and include exemplary embodiments of the present invention and illustrate various objects and features thereof.

Brief Description of the Drawings

FIG. 1 is the HPLC chromatoagram of a radiopharmaceutical preparation in the absence of an oxidant. HPLC was performed approximately six hours after reconstitution.

FIG. 2 is the HPLC chromatogram of a radiopharmaceutical preparation in the presence of p-aminobenzoic acid. HPLC was performed approximately six hours after reconstitution.

FIG. 3 is the HPLC chromatogram of a radiopharmaceutical preparation in the presence of an oxidant of the invention. HPLC was performed approximately six hours after reconstitution.

Detailed Description of the Invention

As required, detailed embodiments of the present invention are disclosed herein; however, it is to be understood that the disclosed embodiments are merely exemplary of the invention that may be embodied in various forms. The figures are not necessarily to scale, and some features may be exaggerated to show details of particular components. Therefore, specific structural and functional details disclosed herein are not to be interpreted as limiting, but merely as a basis for the claims and as a representative basis for teaching one skilled in the art to variously employ the present invention.

Compounds are described that stabilize radiopharmaceutical formulations by inhibiting degradation of the formulations due to further reduction of reducible moieties on the chelating or complexing ligand or further reduction of the central radioactive metal ion. It is further described that radiochemical purity (RCP) in a radiopharmaceutical product is greater than 90% for at least six hours (time of utilization of most of $^{99m}Tc$ radiopharmaceuticals), and the product includes as few impurities as possible. As a result, the radiopharmaceutical formulations containing the stabilizing compounds of the invention show improved stability and reduced levels of impurities, permitting RCPs of greater than 90% after six hours.

As used herein, "radiochemical purity" or "RCP" is determined as follows:

$$RCP = (100-M)(C/100)$$

where

M = % of radiocolloid, as detennined by paper chromatography
C = % radiopharmaceutical, as determined by HPLC

In another aspect, a radiopharmaceutical is described having a reduced impurity content. Preferably, radiopharmaceutical compositions described herein have an impurity content of less than 6%, more preferably less than 4%, and most preferably less than 2%. For example, in FIG. 1, FIG. 2, and FIG. 3. "hydrophilic impurities" are identified to the left of the radiopharmaceutical peak, and "hydrophobic impurities" are identified to the right of the radiopharmaceutical peak, in addition to the impurities contributed by the starting materials.

The present invention preferably uses a quinone to prevent further reduction of the radiopharmaceutical. As used herein, the term "quinone" refers to both unsubstituted and substituted quinones of the Following general structure:

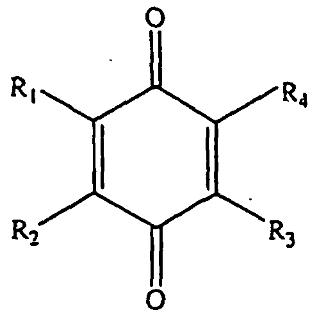

or

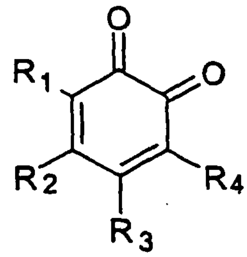

where $R_1$, $R_2$, $R_3$, and $R_4$ are independently selected from hydrogen, hydroxyl, halogen, methyl alkoxy, aryl (including substituted and unsubstituted aryl), heteroaryl, $NO_2$ and

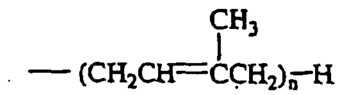

where n is an integer from 0 to 12; or where $R_1$ and $R_2$ taken together form a ring (either substituted or unsubstituted), and $R_3$ and $R_4$ are as defined above. For example, $R_1$ and $R_2$ taken together may form a fused ring comprising five to seven carbon atoms, preferably comprising six carbon atoms. The fused ring may be saturated, unsaturated, or aromatic. Preferably, the fused ring is an aromatic ring. For example, when $R_1$ and $R_2$ taken together form a benzene ring, and $R_3$ and $R_4$ are each hydrogen, the oxidant of the invention may be one of the following:

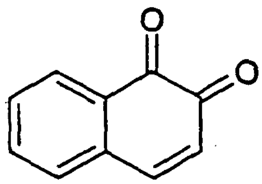

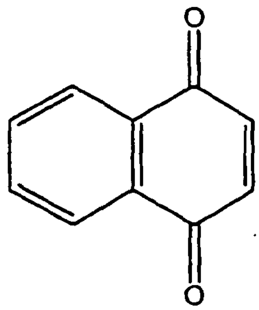

1,2-Naphthoquinone

1,4-Naphthoquinone

A preferred class of quinones used in the present invention are ubiquinones, or coenzymes Q, of the following structure:

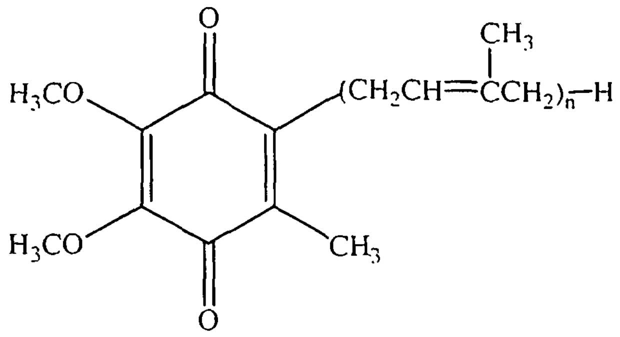

where n is an integer from 0 to 12.

Preferred oxidants include benzoquinone, CAS Registry No. 106-51-4, and coenzyme $Q_0$, with coenzyme $Q_0$ being the most preferred. Benzoquinone (or "2,5-cyclohexadiene-1,4-dione" or "*p*-quinone" or "1,4-benzoquinone" or 1,4-cyclohexadienedione") and coenzyme $Q_0$ have the following chemical structures:

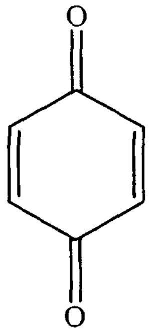

Benzoquinone

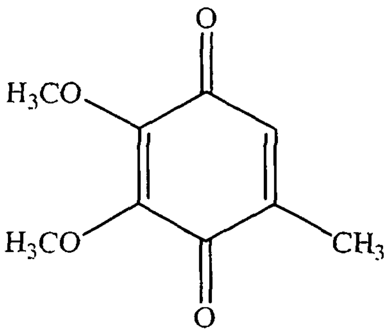

Coenzyme $Q_0$

Preferred oxidants of the present invention may be added either prior to complexation or after formation of the radiopharmaceutical. According to the invention, an oxidant may be used alone or in combination with one or more other oxidants. An artisan skilled in the radiopharmaceutical field will be able to determine by routine experimentation, following the teachings herein, the optimal type(s) of oxidant, the amount, etc., used for a given application. *e.g.*, for a given complexing ligand. For example, the oxidant should be present in sufficient concentration to oxidize any reductant that remains after complexation. As taught herein, where the reductant is stannous, the ratio of stannous to oxidant may range from 1:0.39 to 1:25.

Furthermore, certain oxidants (such as iodine) sublime, and thus are not useful in freeze dried or lyophilized formulations, which are most commercially useful due to *e.g.*, longer shelf life. Oxidants that sublime are thus not useful for manufacturing kits for commercial use because of the reduced shelf life of these formulations. In particular, non-lyophilized formulations may undergo hydrolysis, and are accordingly less desirable than lyophilized formulations.

The present invention can be applied to a wide variety of radiopharmaceuticals that are formed by reduction of a radioactive metal using an excess of reducing agent in the presence of a chelating or complexing ligand, and wherein further reduction of either:

a. the central radioactive metal ion, or
b. a reducible moiety on the chelating or complexing ligand can take place after the formation of the complex.

Radiopharmaceuticals useful in the invention include those that can be prepared by a reaction with a reducing agent able to reduce the metal in an oxidized state to a reduced state that can coordinate with the desired ligand, such as a stannous source, sodium borohydride, Cu(I) salts, formamidine sulphinic acid and the like. Thus, radiopharmaceuticals based, for example, on the following radionuclides $^{99m}$Tc (and other isotopes of Tc), $^{51}$Cr, $^{67}$Cu, $^{97}$Ru, $^{188}$Re, $^{186}$Re, and $^{199}$Au are useful in the invention. $^{188}$Re, $^{186}$Re and $^{99m}$Tc isotopes are preferred.

The oxidants of the invention may be used to stabilize radiopharmaceutical formulations in which reaction with excess reductant after the desired compound has formed would otherwise cause the central metal to be reduced from the desired oxidation state (e.g. Tc or Re(V)) to a lower one (e.g. Tc or Re (IV or III)). For example, oxidants of the invention may be used to prevent reduction of Tc(V) (in for example Tc-MAG3 or Tc- phosphine based compounds) to a lower oxidation state (such as Te(IV) in the case of Tc-MAG3 or Tc(III) or Tc(I) in the case of Tc-phosphine compounds).

Additionally, the present invention is useful to stabilize radiopharmaceutical formulations in which excess reductant present after the desired compound has been formed would otherwise cause a readily reducible group on the chelating or complexing ligand to be further reduced.

Thus the oxidants of the invention may be used with radiopharmaceuticals containing a wide variety of chelating or complexing ligands to inhibit degradation due to further reduction of reducible moieties on such ligands. Many ligands that bind to radionuclide metals are tetradentate and contain a combination of four nitrogen and/or sulfur metal coordinating atoms (i.e. N4, N3S, N2S2 and the like). For example, N4 chelators are described in U.S. Patent Nos. 6,093,382, 5,608,110; 5,665,329; 5,656,54; and 5,688,487. Certain $N_3S$ chelators are described in PCT/CA94/00395 and in PCT/CA94/00479. The invention may apply to derivatives of the complexing ligand mercapto-acetyl-acetyl-glycyl-glycine (MAG3), which contains an N3S, and to N2S2 systems such as MAMA, DADS. CODADS and the like. These ligand systems and a variety of others are described in Liu and Edwards. Chem Rev. 1999, 99,2235-2268 and references therein.

This invention is also potentially applicable to complexes containing ligand atoms which are not donated to the metal in a tetradentate array. These include the boronic acid adducts of technetium and rhenium dioximes, such as are described in U.S. Patent Nos. 5,183,653; 5,387,409; and 5,118,797.

The possible reducible groups present in the ligand molecule may include, for example: aldehydes, ketones, α-substituted lactones, conjugated ketones, α-substituted esters, sugar lactones, cyclic anhydrides, N-alkylphthalimides, imines and iminiuin salts, aromatic nitro groups, nitrosamines, quinones, sulfenyl chlorides, 1.2-diketoethylenes, naphthoquinones, o-alkyl dioxaimines, catechols, and ortho phenylenediamines.

Such reducible groups may also be heterocyclic groups less resistant to the reduction conditions due to the presence of an electron withdrawing substituent, like a nitro group. This may occur, for example, in imaging agents that bear a nitroimidazole group on the complexing ligand. The nitroimidazole moiety in these agents is used to target the radiolabeled compound to hypoxic tissue (see for example H.J. Machulla, Imaging of Hypoxia, Vol.33, Developments in Nuclear Medicine, Kluver Publ., 1999). Further reduction of this targeting group may remove such targeting ability.

In a second example, the reaction of excess reducing agent with a disulfide bond in a protein or in a ligand that bears a cyclic peptide may cause this disulfide bond to be reduced to yield two free thiols, opening the cyclic ring and potentially abolishing the ability of such protein or cyclic peptide to bind to its target. See for example Thakur et al, J. Labelled Compounds and Radiopharmaceuticals 1993, Vol. 32, p.365-367.

The invention is of particular utility when there is an imidazole moiety present on the ligand molecule, and more particularly when a nitro group is present in the cycle, as described in U.S. Patent No. 5,665,329; Su, Zi-Fen et al, Bioconjugate Chem. (2000), 11(5), 652-663: WO-A-0043004; Zhang. Xiuguo et al., Bioconjugate Chem. (2000), 11(3), 401-407.

It should be understood that the foregoing ligand systems are provided only as examples, and that modifications or alternatives that are equivalent to it within the spirit and scope of the invention are envisaged.

In a preferred embodiment of the present invention, added to radiopharmaceutical the oxidant has the following structure:

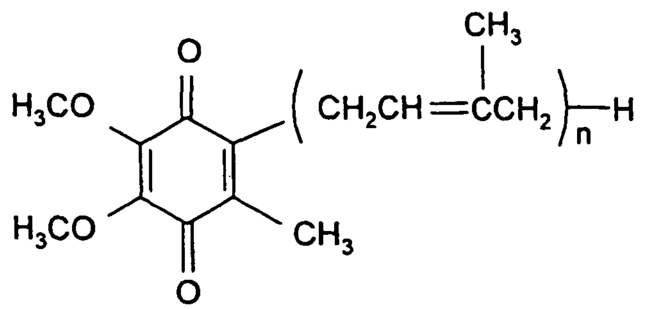

where n is an integer from 0 to 12, in particular, wherein the oxidant is coenzyme $Q_0$, in particular, wherein the radiopharmaceutical comprises a compound of formula I:

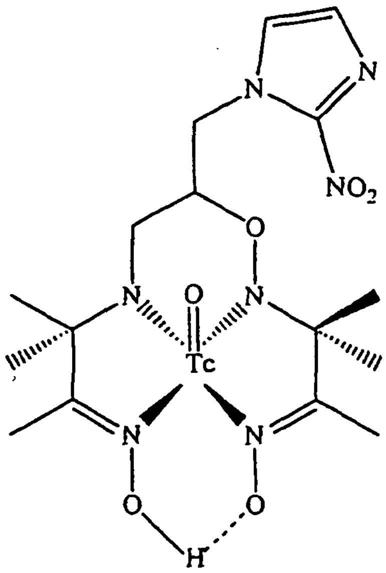

Formula I

This compound localizes preferentially in hypoxic tissue and has potential applications in pathologies characterized by low oxygen content such as identification of viable versus necrotic tissue Following myocardial infarction or stroke and the detection of hypoxic tumors. The nitroimidazole group is responsible for the hypoxic tropism of the compound of Formula I. Nitroimidazoles are normally reduced in all viable cells, but in the absence of an adequate supply of oxygen they undergo further reduction to more reactive products that bind to cell components (see, for example, Edwards DI: Nitroimidazoles drugs-action and resistance mechanism. I. Mechanism of action. J. Antimicrob. Chemother. 1993, 31, 9-20).

The proposed multi-step reduction pathway for nitroimidazoles in the cell is as follows:

$$RNO_2 \xleftrightarrow{+1e-} RNO_2^{\bullet -} \xrightarrow{+1e-} RNO \xrightarrow{+2e-} RNHOH \xrightarrow{+2e-} RNH_2$$

The oxidants of the invention may be used to improve the stability of formulations having a compound of Formula I. Indeed, as shown in more detail in Example 2 below, use of an oxidant of the present invention with formulations having a compound of Formula I, whether added before or after complexation, stabilized the radiopharmaceutical preparation, eliminating a lipophilic impurity caused by reduction of the nitroimidazole group and permitting RCPs of well over 90% at six hours. Use of benzoquinone, CAS Registry No.106-51-4, and coenenzyme $Q_0$ as stabilizers in formulations having a compound of Formula I gave RCPs ranging from 94-96% at six hours.

The present invention may also provide stabilized formulations of radiopharmaceuticals containing an oxidant of the invention as well as the appropriate and usual additives such as buffers, bulking agents, etc. In another embodiment, the present invention may also include single and/or multi-dose kits for preparing formulations of radiopharmaceuticals containing a stabilizing oxidant of the invention. Several potential kit formulations are envisioned. For example, the complexing ligand and oxidant may be present in one vial while the stannous or other reducing source may be present in a second vial. In another example, the kit formulation may include the complexing ligand in one vial, the stannous source in a second vial and the oxidant in a third vial. In yet another example, the kit may include a transfer (or trans-chelating) ligand and stannous or other reducing agent in the first vial and the complexing ligand and oxidant in the second vial. Similarly, in an additional kit formulation, a transfer ligand and stannous or other reducing agent are present in one vial, the complexing ligand is in a second vial and the oxidant is present in a third vial. In a further embodiment, the kit formulations may include the usual additives and bulking agents known to those skilled in the art. Methods of reconstituting the kits with radioisotopes arc well known to those skilled in the art.

Kits of the present invention comprise one or more vials containing the sterile formulation of a predetermined amount of a complexing ligand, an oxidant and optionally other components such as reducing agents, transfer ligands, buffers, lyophilization aids or bulking agents, stabilization aids, solubilization aids and bacteriostats. The inclusion of one or more optional components in the formulation will frequently improve the ease of synthesis of the radiopharmaceutical by the practicing end user, the ease of manufacturing the kit, the shelf-life of the kit, or the stability and shelf-life of the radiopharmaceutical. The improvement achieved by the inclusion of an optional component in the formulation must be weighed against the added complexity of the formulation and added cost to manufacture the kit. The one or more vials that contain all or part of the formulation can independently be in the form of a sterile solution or a lyophilized solid.

Buffers useful in the preparation of radiopharmaceuticals and in diagnostic kits useful for the preparation of the radiopharmaceuticals include but are not limited to phosphate, citrate, sulfosalicylate, and acetate. A more complete list can be found in the United States Pharmacopeia.

Lyophilization aids or bulking agents useful in the preparation of diagnostic kits useful for the preparation of radiopharmaceuticals are known in the art and include lactose, sodium chloride, maltose, sucrose, PEG 8000, cyclodextrins, such as hydroxypropyl-γ-cyclodextrin (HP-γ-CD), dextran, Ficoll, and polyvinylpyrrolidine (PVP). Of these, sodium chloride, maltose, sucrose, PEG 8000, HP-γ-CD, and dextran are preferred bulking agents for use with the invention, with maltose, sucrose, and H.P-γ-CD being the most preferred.

Stabilization aids useful in the preparation of radiopharmaceuticals and in diagnostic kits useful for the preparation of the radiopharmaceuticals include but are not limited to ascorbic acid, *para*-aminobenzoic acid (PABA), cysteine, monothioglyccrol, sodium bisulfite, sodium metabisulfite, gentisic acid, and inositol.

Solubilization aids useful in the preparation of radiopharmaceuticals and in diagnostic kits useful for the preparation of the radiopharmaceuticals include but are not limited to ethanol, glycerin, polyethylene glycol, propylene glycol, polyoxyethylene sorbitan monooleate, sorbitan monooloeate, polysorbates, poly(oxyethylene)poly(oxypropylene)poly (oxyethylene) block copolymers (Pluronics) and lecithin. Preferred solubilizing aids are polyethylene glycol, and Pluronics.

Bacteriostats useful in the preparation of radiopharmaceuticals and in diagnostic kits useful for the preparation of the radiopharmaceuticals include but are not limited to benzyl alcohol, benzalkonium chloride, chlorbutanol, and methyl, propyl or butyl paraben.

In a preferred embodiment, the kits for the preparation of a metal radionuclide-containing radiopharmaceutical further comprises a transfer ligand, a bulking agent, a stabilization aid, a solubilization aid or a bacteriostat, in particular, wherein the bulking agent is selected from mattose, sucrose, and hydroxypropyl-γ-cyclodextrin.

A component in a diagnostic kit can also serve more than one function. A reducing agent can also serve as a stabilization aid, a buffer can also serve as a transfer ligand, a lyophilization aid can also serve as a transfer, ancillary or co-ligand and so forth.

The predetermined amounts of each component in the formulation are determined by a variety of considerations familiar to those skilled in the art. These considerations are in some cases specific for that component and in other cases dependent on the amount of another component or the presence and amount of an optional component. In general, the minimal amount of each component is used that will give the desired effect of the formulation. The desired effect of the formulation is that the practicing end user can synthesize the radiopharmaceutical and have a high degree of certainty that the radiopharmaceutical can be safely injected into a patient and will provide diagnostic information about the disease state of that patient.

<u>Examples</u>

Example 1

In a preferred embodiment of the present invention, oxidants of the invention are added to a compound of Formula I, a $^{99in}$Tc(V)-diamine dioxime complex that contains a 2-nitroimidazole substituent:

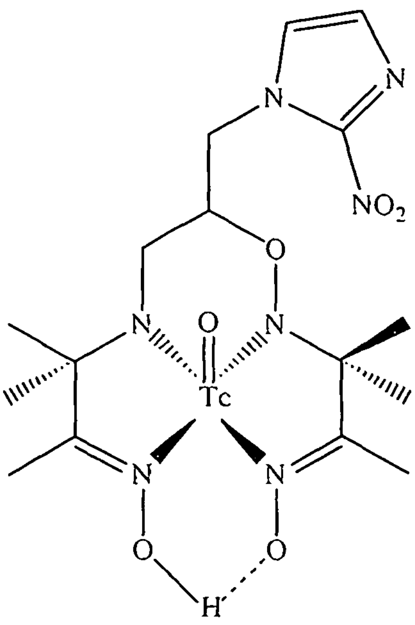

Formula I

1. Preparation of a Complexing Ligand for the compound of Formula I The structure of the complexing ligand of Formula II is as follows:

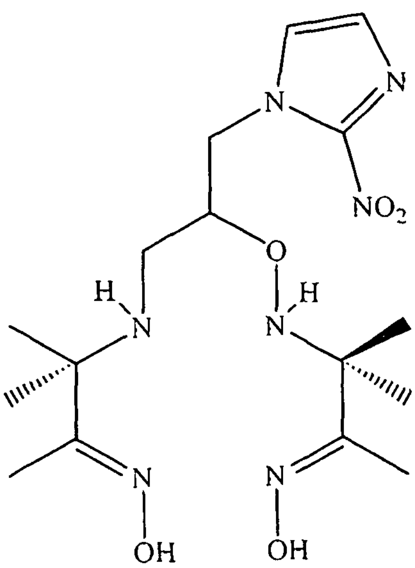

Formula II

This ligand, which complexes with $^{99m}$Tc to form the compound of Formula I, may be prepared as set forth below. Additionally, methods for the preparation of various complexing ligands, including the Compound I complexing ligand, are set forth in U.S. Patent No. 5.608,110, the disclosure of which is incorporated by reference herein in its entirety:

A. Preparation of 2-[2-Hydroxy-2-(nitro-1*H*-imidazole-1-yl) ethyl]-1*H*-isoindole-1,3(2H)-dione.

To a solution of N-(2,3-epoxypropyl)phthalimide (commercially available, 25.0 g, 0.123 mol) in ethanol (200 mL) 2-nitroimidazole (commercially available, 14.5 g, 0.128 mol) and potassium carbonate (1.5 g) were added and the reaction mixture was heated under reflux for about 6 hours during which time a light yellow solid formed. The reaction mixture was cooled and the yellow solid obtained was filtered and dried. m.p. 213-14°C.

B. Preparation of a-(Aminoimethyl)-2-nitro-1*H*-imidazole-1-ethanol.

To a suspension of 2-[2-hydroxy-2-(nitro-1*H*-imidazol-1-yl)ethyl]-1H-isoindole-1,3(2H)-dione from Step A. (43.3 g, 0.137 mol) in methanol (400 mL), anhydrous hydrazine (5.5 g, 0.172 mol) was added and the mixture was refluxed for about 8 hours. A clear solution was obtained after 1 hourand light yellow solid began to separate after 3 hours. The reaction mixture was cooled and the methanol was removed on a rotary evaporator.

C.Preparation of a-[(t-Boc-amino)methyl]-2-nitro-1*H*-imidazole-1-ethanol.

The light yellow solid obtained from Step B was treated with sodium carbonate (31.8 g. 0.3 mol) in water (200 mL). Tehahydrofuran [THF.] (800 mL) was added to this mixture and cooled to about 0°C. Di-t-butyl dicarbonate (43.6 g, 0.2 mol) was added to the THF-water mixture and stirred at about 0°C for 1 hour and RT for 72 hours. THF-water was removed on a rotary evaporator and the residue was extracted with ethyl acetate (3 x 150 mL). The ethyl acetate layer was washed with water dried over sodium sulphate ($Na_2SO_4$) and evaporated on a rotary evaporator to yield the title compound as a yellow solid. The yellow solid obtained was triturated with ether (200 mL) and filtered. It was recrystallized from hexane-ethyl acetate. m.p. 128-29°C.

D. Preparation of N-[1-[(t-Boc-amino)methyl]-2-(2-nitro-1*H*-imidazol-1-yl)ethoxy)phthalimide.

N-hydroxyphthalimide (18.0 g, 0.11 mol), a-[*t*-Boc-amino)methyl]-2-ntro-*1H*-imidazole-1-ethanol (28.6 g, 0.1 mol) ), and triphenylphosphine (28.85 g, 0.1 mol), were dissolved in THF (200 mL), and treated with diethyl azodicarboxylate (21.75 g, 0.125 mol). The reaction mixture became dark red and the color disappeared after a few minutes. A slight exothermic reaction was observed during the addition of diethyl azodicarboxylate. The reaction mixture was stirred at room temperature for about 24 hours, and evaporated on a rotary evaporator to dryness. The residue was dissolved in ether (300 mL), and the solid that formed was filtered and silica gel (100 g) was added to the filtrate. The solvent was evaporated on a rotary evaporator to afford a free flowing powder. The free flowing powder was loaded on to a silica gel column (76 x 560 mm. 1.5 Kg) and eluted first with hexane-ethyl acetate 7:3, followed by hexane-ethyl acetate 3:7. Fractions containing the product were collected and evaporated to give the product as a white foam.

E. Preparation of 1-[2-(Aminooxy)-3-1-Boc-amino)propyl]-2-nitro-1*H*-imidazole.

Anhydrous hydrazine (2.0 g, 0.062 mol) was added to a solution of 2-[1[(*t*-Boc-amino)methyl]-2-(2-nitro-1*H*-imidazol-1-yl)ethoxy] *1H*-isoindole-1.3(2H)-dione (24.0 g. 0.056 mol) in methylene chloride (150 mL) and the mixture was stirred at room temperature for about 6 hours. A light yellow solid (Phthalyl hydrazide) began to Form within 10 min. The solid, which formed, was filtered and the filtrate was evaporated on a rotary evaporator. The thick oil obtained was triturated with ether (200 mL) and the resultant precipitate was filtered and dried under vacuum. Yield: 15.8 g (94%). This was used in the next step without further purification. A portion of the amine was recrystallized from hexane-ethyl acetate, m.p. 95-96°C.

F. Preparation of 1-[3-Amino-2-(aminooxy)propyl-2-nitro-1*H*-imidazole dihydrochloride.

Methanolic HCl (30 mL) was added to a solution of 1-[2-(aminooxy)-3-(*t*-Boc-amino)propyl]-2-nitro-1*H*-imidazole (15.6 g. 0.052 mol) in methanol (15 mL), and the mixture was stirred at room temperature for about 1 hour. Ether (200 mL) was added to the methanolic solution and the 1-[3-amino-2-(aminooxy)propyl]-2-nitro-1*H*-imidazole dihydrochloride, which formed, was filtered and dried under vacuum. Yield 12.9 g (91 %). A portion of the hydrochloride was crystallized from methanol/ether. m.p. 143-44°C dec.

G. Preparation of 3-chloro-3-methyl-2-nitrosobutane.

In a 500-mL 3-necked flask fitted with a mechanical stirrer,a dropping funnel and a thermometer was placed 2-methyl-2-butene (91.4 mL, 0.846 mol) at RT and, with stirring, isoamyl nitrite (107.4 mL. 0.8 mol) was introduced. The

mixture was then cooled to about -15°C and concentrated HCl (89.5 mL, 0.907 mol) was added dropwise over a period of about 40 min maintaining the temperature at -10°C to ~-5°C, giving a light blue slurry. The slurry was stirred at -10°C to ~-5°C for an additional 30 min. The pasty mass was filtered and washed with cold -20°C petroleum ether. The wet product was dissolved in petroleum ether (b.p. 30-60°C, 400 mL) and the solution was decanted to remove water and dried ($Na_2SO_4$). The solution was filtered and cooled to -50°C with occasional stirring, and the white crystalline solid was collected by filtration, washed with cold (-50°C) petroleum ether and dried under vacuum at RT overnight. m.p. 72-74°C.

H. Preparation of the Compound 1 Complexing Ligand; [3,3,9,9-Tetramethyl-6[(2-nitro-1H-imidazol-1-yl)methyl]-5-oxa-4,8-diazaundecane-2,10-dione dioxime]

In a nitrogen-flushed, 500 mL round bottomed flask equipped with a magnetic stirrer were placed 1-[3-Amino-2-(aminooxy)propyl]-2-nitro-*1H*-imidazole dihydrochloridc (12.74 g. 0.0465 mol), acetonitrile (150 mL), and N, N'-diisopropyl ethylamine (32.4 g, 43.7 mL, 0.25 mol). 3-Chloro-3-methyl-2-nitrosobutane (14.9 g, 0.11mol) was added to this mixture and stirred at room temperature for 12 hours. (When the 3-chloro-3-methyl-2-nitrosobutane was added, the reaction mixture became slightly exothermic). Acetonitrile was removed on a rotary evaporator and the thick oil obtained was basified with saturated potassium carbonate solution (25 g, in 25 mL of water). The light green oil obtained was extracted with ethyl acetate and dried over $Na_2SO_4$. Ethyl acetate was removed on a rotary evaporator and the oil obtained was dried under vacuum to afford a foamy solid. The foamy solid was dissolved in acetonitrile (200 mL) and left at room temperature for about 2 hours. The solid that formed was filtered and recrystallized from acetonitrile.m.p. 170-171°C.

For the experiments set forth below, the complexing ligand was used in the form of a freeze-dried formulation containing 2 mg of the complexing ligand of Formula II.

2. Preparation of the Compound of Formula I without Oxidants of the Invention

The compound of Formula I was prepared in the absence of oxidants of the invention using a freeze-dried formulation containing the complexing ligand discussed above, and one of two different commercially available kits for the preparation of $^{99m}$TcDTPA (Techneplex-DTPA kit and Draximage-DTPA kit) as the stannous source. In the first preparation (Preparation 1), a freeze-dried formulation containing 2 mg of the complexing ligand of Formula II was dissolved in a sufficient amount of $^{99m}TcO_4^-$ generator eluent to reach an activity of about 40 mCi. To this solution was added a sufficient amount of saline to bring the volume to 2.0 mL, followed by 0.5 mL of the Techneplex-DTPA (17.5 $\mu$g of $SnCl_2$) kit (reconstituted with 10 mL of saline).

In the second preparation (Preparation 2), a freeze dried formulation containing 2 mg of the complexing ligand of Formula II was dissolved in a sufficient amount of $^{99m}TcO_4^-$ generator eluent to reach an activity of about 40 mCi. To this solution was added a sufficient amount of saline to bring the volume to 2.0 mL, followed by 0.5 mL of the Draximage-DTPA (17.5 $\mu$g of $SnCl_2$) kit (reconstituted with 10 mL of saline).

Aliquots of Preparations I and 2 were withdrawn after about six hours and analyzed by HPLC. The compound was eluted using the following conditions: isocratic elution, 45% $CH_3CN$ / 55% 0.1 M ammonium acetate pH 4.0, flow rate about 1 mL/min, YMC Basic S5 4.6 X 250 mm column.

Paper chromatography was employed to determine the quantity of radiocolloid present in each kit using Gelman solvent saturator pads developed in 50:50 methanoksaline. In this system, the percentage of activity retained at the origin corresponds to the % of radiocolloid.

Using Preparation 1 of the compound of Formula I, RCP's of greater than 90% at six hours were not obtained. The HPLC chromatogram shown in FIG. 1 illustrates the composition of Preparation 1 (reconstituted from a kit using Techneplex-DTPA as the stannous source at six hours post reconstitution). The side products formed are mostly hydrophilic species with a shorter retention time (2.8, 3.6 and 4 min) than the compound of Formula I, which elutes at 5.7 min. The peak eluting at 2.8 min. was identified as TcDTPA, the peak at 3.6 min. as $TcO_4^-$, while the peak at 4 min. may be a reduced form of the compound of Formula I. An unidentified lipophilic peak eluting at 6.9 min that accounts for up to 3% of the radioactivity is also present.

The HPLC chromatogram shown in FIG. 2 illustrates that when a different commercially available Sn-DTPA source, Draximage-DTPA, was used to prepare the compound of Formula I (Preparation 2), the RCP increased to about 90% at six hours but the unidentified lipophilic peak was still present.

The only difference between the Techneplex-DTPA and the Draximage-DTPA kit formulations is the presence of p-aminobenzoic acid (PABA) in the latter one. PABA is a known radical scavenger (Hu, Miao Lin; Chen Ling-Chun; Sano, Mitsuaki: Para-aminobenzoic acid scavenges reactive oxygen species and protects DNA against UV and free radical damage. J. Nutr. Biochem. 1995, 6(9) 504-8) and appears to be able to neutralize the radical anion $NO_2^-$, the first step in the reduction cascade of the nitro group.

It is believed that the excess of stannous present after the reduction of the pertechnetate, together with the radicals generated by radiolysis, were responsible for the reduction of the nitroimidazole moiety on the compound of Formula I, leading to the formation of both hydrophilic and lipophilic impurities over time.

3. Preparation of the Compound of Formula I with Oxidants of the Invention

To inhibit this degradation, the compound of Formula 1 was formulated with a number of different oxidants. Except as indicated in Table 1, the oxidants were added before complexation of $^{99m}Tc$ with the complexing ligand of Formula II. Thus, sodium hypochlorite and Benzoquinone, CAS Registry No. 106-51-4, were added before complexation in some formulations and after complexation in other formulations. However, the remaining oxidants tested (Coenzyme Q0, iodine, $KMnO_4$. $CuCl_2$, CuBr and $FeCl_3$) were all added prior to complexation.

The procedure for addition of the oxidants before the complexation is illustrated with Benzoquinone, CAS Registry No. 106-51-4:

A freeze-dried formulation containing 2 mg of the complexing ligand of Formula II was dissolved in a sufficient amount of $^{99m}TcO_4^-$ generator eluent to yield an activity of about 40 mCi. To this solution was added in the following order: 135 µL of a a 1.15 mM solution of benzoquinone, sufficient saline to bring the volume to 2.0 mL, and 0.5 mL of a Draximage-DTPA (17.5 µg of $SnCl_2$) kit (reconstituted with 10 mL of saline).

The procedure for addition of the oxidants after complexation is also illustrated with benzoquinone:

A freeze-dried formulation containing 2 mg of the complexing ligand of Formula II was dissolved in a sufficient amount of $^{99m}TcO_4^-$ generator eluent to reach an activity of about 40 mCi. To this solution was added sufficient saline to bring the volume to 2.0 mL, followed by 0.5 ml of the Draximage-DTPA (17.5 µg of $SnCl_2$) kit (reconstituted with 10 mL of saline). Fifteen minutes later, 135 µL of a 1.15 mM solution of benzoquinone was added.

4. Testing Formulations of the Compound of Formula I with Oxidants of the Invention

Formulations of the compound of Formula I containing oxidants of the invention were assessed for RCP (radiochemical purity) by HPLC at 15 minutes and 6 hours using the procedure described above. Many oxidants, organic and inorganic, with potentials ranging from 1.358 to 0.341 volts (relative to the standard hydrogen electrode), were tested. Although these oxidants were able to oxidize $Sn^{2+}$, many of them still allowed complex formation when added prior to stannous addition. The results obtained with the various formulations of Compound 1 and different oxidants are summarized in Table 1 below.

As shown in Table 1. strong oxidants, such as sodium hypochlorite, must be added after complex formation. Table 1 illustrates that the RCP of formulations of the compound of Formula I containing oxidants was well over 90% at six hours and the lipophilic impurity eluting at 6.9 min was completely eliminated.

Table 1: Effect of oxidants on Radiochemical Purity (RCP) of Compound 1

| Oxidant | Ratio of Sn: Oxidant | Amount of Oxidant | 15 min. RCP | 6 hr. RCP | Lipophilic peak 6 hr. |
|---|---|---|---|---|---|
| Benzoquinone | 1:1 | 8.4 µg | | 96.2 | Not detectable |
| Benzoquinone | 1:2 | 16.8 µg | | 96.2 | Not detectable |
| Benzoquinone | 1:2 | 16.8 µg | 98.2 | 96 | Not detectable |
| Benzoquinone | 1:2 | 16.8 µg | 98 | 95.7 | Not detectable |
| Benzoquinone | 1:1 | 8.4 µg | 96.5 | 96.5 | Not detectable |
| Benzoquinone | 1:0.7 | 5.9 µg | 97.9 | 96.2 | Not detectable |
| Benzoquinone | 1:1.4 | 11.8 µg | 98 | 96.3 | Not detectable |
| Benzoquinone | 1:3.5 | 29.4 µg | 97.8 | 95.8 | Not detectable |
| Benzoquinone | 1:5 | 42 µg | 97.3 | 95 | Not detectable |
| Benzoquinone after addition of Draximage DTPA | 1:10 | 84 µg | 97.3 | 94.2 | Not detectable |

(continued)

| Oxidant | Ratio of Sn: Oxidant | Amount of Oxidant | 15 min. RCP | 6 hr. RCP | Lipophilic peak 6 hr. |
|---|---|---|---|---|---|
| Benzoquinone after addition of Draximage DTPA | 1:15 | 126 μg | 96.7 | 93.5 | Not detectable |
| Coenzyme Q0 | 1:25 | 353 μg | 95.7 | 94.7 | Not detectable |
| Coenzyme Q0 | 1:25 | 353 μg | 93 | 91.2 | Not detectable |
| Coenzyme Q0 | 1:25 | 353 μg | 95.9 | 93.1 | Not detectable |
| Tetrahydroxy benzoquinone | 1:2 | 26.7 μg | 95.6 | 87.3 | 1.1 |
| Iodine | 1:1.4 | 27 ug | 97.9 | 95.3 | Not detectable |
| Iodine | 3.4 | 67 ug | 96.5 | 93.3 | Not detectable |
| $KMnO_4$ | 1:0.39 | 7 μg | 93.3 | 89 | 1.9 |
| $KMnO_4$ | 1:1 | 18 μg | 97.6 | 92.9 | 1.6 |
| $KMnO_4$ | 1:2 | 37 μg | 97.6 | 92.2 | 2.3 |
| $KMnO_4$ | 1:3 | 55 μg | 97.4 | 91.2 | 3 |
| Sodium hypochlorite | 1:2.4 | 12 μg of $Cl_2$ | 10 | | Not detectable |
| Sodium hypochlorite | 1:1 | 5 μg of $Cl_2$ | 49.5 | | Not detectable |
| Sodium hypochlorite after ddition DraximageDTPA | 1:1 | 5 μg of $Cl_2$ | 97 | 92.6 | Not detectable |
| Sodium hypochlorite after addition of DraximageDTPA | 1:1 | 5 μg of $Cl_2$ | 96.8 | 93.4 | Not detectable |
| Sodium hypochlorite after addition of DraximageDTPA | 1:2 | 10 μg of $Cl_2$ | 97.4 | 93.4 | Not detectable |
| Sodium hypochlorite after adddition of DraximageDTPA | 1:5 | 25 μg of $Cl_2$ | 97.2 | 93.6 | Not detectable |
| $CuCl_2$ | 1: 1 | 13 μg | 97.3 | 93.9 | 1.5 |
| $CuCl_2$ | 1: 2 | 26 μg | 4.3 | | |
| CuBr | 1: 2 | 22 μg | 94.8 | 92.5 | 1 |
| CuBr | 1: 3 | 33 μg | 98 | 94 | 0.8 |
| $FeCl_3$ | 1: 2 | 84 μg | 95.5 | 91.1 | 1.2 |

The percentage of $^{99m}$Tc-radiocolloid, which is not detectable by HPLC, was determined by paper chromatography, as described above. The percentages were in the normal average (0.5 - 1.0 %) for all the reconstituted kits.

FIG. 3 is the HPLC chromatogram of a formulation of the compound of Formula I containing benzoquinone, CAS Registry No. 106-51-4, at 6 hours post reconstitution. This figure illustrates the improvement achieved with the use

of benzoquinone: the lipophilic peak is not present and the unidentified hydrophilic impurities are below 1%.

Example 2

The next step of this study was to freeze dry different formulations of the compound of Formula I containing coenzyme $Q_0$, a preferred oxidant of the invention, and one of several bulking agents. The introduction of a bulking agent is necessary to form a visible and stable pellet at the bottom of the vial.

Coenzyme $Q_0$ was selected for use in this experiment because it is more suitable for a freeze drying process than either iodine, which can easily sublime in the process, or sodium hypochlorite, which was rejected because, being a very strong oxidizer, it has to be introduced after complex formation and thus requires a three vial formulation.

Four formulations containing 4 mg of the complexing ligand of Formula II. 0.7 mg of coenzyme $Q_0$ and 10 mg of one of several bulking agents were prepared and freeze dried. Specifically, the complexing ligand of Formula II was dissolved in 90 ml oF a pH 2.1 HCl solution and the pH was adjusted to 8.2 using 0.1 N NaOH. 35.4 mg of coenzyme $Q_0$ (dissolved in 5 mL of water) was added and the volume was adjusted to 100 mL. This bulk solution was divided in four 25 mL aliquots. 250 mg of the bulking agent was added to each aliquot and the pH was adjusted to 8.2 using 0.001 N NaOH. These solutions were filtered and 2 mL of each formulation was dispensed in vials for the freeze drying process. The freeze-dried formulations were reconstituted with 40 mCi of $^{99m}TcO_4^-$ using Draximage-DTPA as the stannous source, as described in Example I. The formulations were analyzed by HPLC at 15 minutes, 2 hours, 4 hours and 6 hours using the conditions described. The results are summarized in Table 2.

Table 2: RCP Values For Freeze-dried Kits

| Bulking agent | 15 min. | 2 hr. | 4 hr. | 6 hr. | Lipophilic peak 6 hr. |
|---|---|---|---|---|---|
| 20 mg of Sucrose | 97 | 96.7 | 95.3 | 94.6 | Not detectable |
| 20 mg of Dextran | 96.1 | 94.7 | 94 | 93 | Not detectable |
| 20 mg Hydroxypropyl-γ-cyclodextrin | 96.8 | 95.8 | 94.9 | 94.2 | Not delectable |
| 20 mg Maltose | 96.8 | 94.8 | 94.8 | 94.2 | Not detectable |

The percentages of radiocolloid were in the normal average (0.5- 1.0 %) for all the reconstituted kits. All formulations tested had an RCP greater than 90% at six hours after reconstitution when reconstituted with 40 mCi of $^{99m}Tc$.

## Claims

1. A kit for preparing a metal radionuclide-containing radiopharmaceutical, the kit comprising a predetermined quantity of a complexing ligand and a predetermined quantity of an oxidant, wherein the oxidant has the following structure:

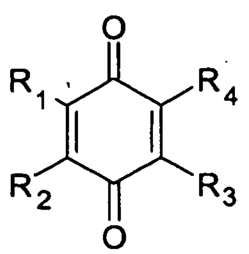

or

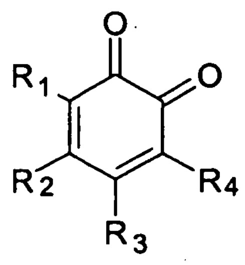

where $R_1$, $R_2$, $R_3$, and $R_4$ are independently selected from hydrogen, hydroxyl, halogen, methyl, alkoxy, aryl, heteroaryl, $NO_2$ and

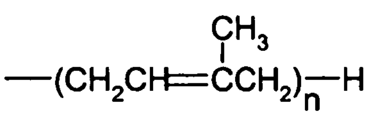

where n is an integer from 0 to 12; or where $R_1$ and $R_2$ taken together form a ring and $R_3$ and $R_4$ are as defined above, and the radiopharmaceutical has a radiochemical purity of greater than 90% at six hours after reconstitution, in particular wherein the radiochemical purity is greater than 92 % at six hours after reconstitution, in particular, wherein the radiochemical purity is greater than 95 % at six hours after reconstitution.

**2.** The kit of claim 1, wherein the oxidant is introduced prior to complex formation.

**3.** The kit of claim 1, wherein the oxidant has the following structure:

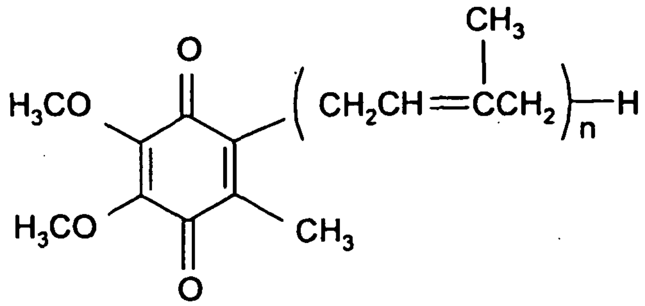

where n is an integer from 0 to 12, in particular, wherein the oxidant is coenzyme $Q_0$.

**4.** The kit of claim 1, wherein the ligand and the oxidant are in one vial.

**5.** The kit of claim 1, wherein the complexing ligand comprises a compound of Formula II:

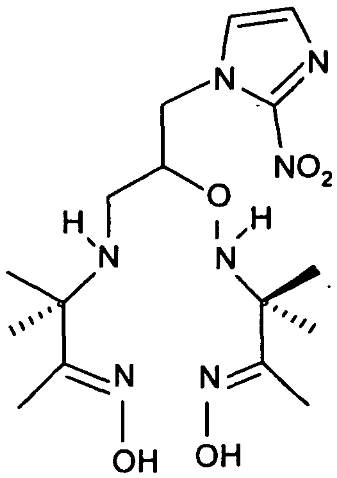

Formula II

**6.** The kit of claim 1, further comprising a reducing agent, in particular, wherein the reducing agent is selected from stannous chloride, stannous pyrophosphate, stannous fluoride, stannous tartrate, stannous glucoheptonate, stannous DTPA, a borohydride salt, sodium dithionite, a Cu(I) salt, and a formamidine sulphinic acid, in particular, wherein the reducing agent is a stannous compound, in particular, wherein the stannous and the oxidant are present in a ratio from 1:0.39 to 1:25.

**7.** The kit of claim 1, wherein the radiopharmaceutical comprises a radionuclide selected from $^{99m}T_c$, $^{51}Cr$, $^{67}Cu$, $^{97}Ru$, $^{188}Re$, $^{186}Re$, and $^{199}Au$.

**8.** The kit of claim 1, further comprising a transfer ligand, a bulking agent, a buffer, a stabilization aid, a solubilization

aid or a bacteriostat, in particular, wherein the bulking agent is selected from maltose, sucrose, and hydroxypropyl-y-cyclodextrin.

**9.** A kit for the preparation of a metal radionuclide-containing radiopharmaceutical, the kit comprising a lyophilized composition comprising a predetermined quantity of a complexing ligand and a predetermined quantity of an oxidant, wherein the oxidant has the following structure:

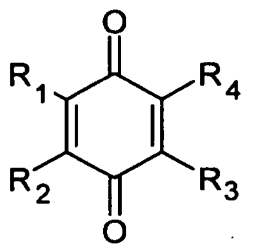

or

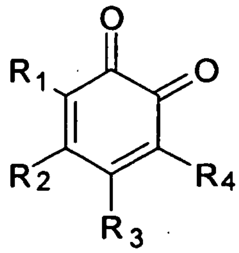

where $R_1$, $R_2$, $R_3$, and $R_4$ are independently selected from hydrogen, hydroxyl, halogen, methyl, alkoxy, aryl, heteroaryl, $NO_2$ and

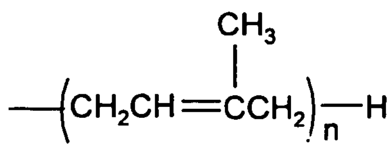

where n is an integer from 0 to 12; or where $R_1$ and $R_2$ taken together form a ring and $R_3$ and $R_4$ are as defined above.

**10.** The kit of claim 9, wherein the radiopharmaceutical has a radiochemical purity of greater than 90% at six hours after reconstitution, in particular, wherein the radiochemical purity is greater than 95% at six hours after reconstitution.

**11.** The kit of claim 9, wherein the ligand and oxidant are in one vial.

**12.** The kit of claim 9, wherein the oxidant has the following structure:

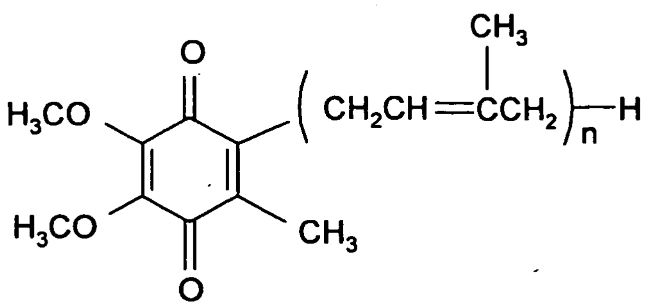

where n is an integer from 0 to 12, in particular, wherein the oxidant is coenzyme $Q_0$.

**13.** The kit of claim 12, wherein the radiochemical purity is greater than 92% at six hours after reconstitution.

**14.** The kit of claim 12, further comprising a reducing agent, in particular, wherein the reducing agent is selected from stannous chloride, stannous pyrophosphate, stannous fluoride, stannous tartrate, stannous glucoheptonate, stannous DTPA, a borohydride salt, sodium dithionite, a Cu(I) salt, and a formamidine sulphinic acid, in particular, wherein the reducing agent is a stannous compound, in particular, wherein the stannous and the oxidant are present in a

ratio from 1:0.39 to 1:25.

**15.** The kit of claim 12, wherein the radiopharmaceutical comprises a radionuclide selected from $^{99m}Tc$, $^{51}Cr$, $^{67}Cu$, $^{97}Ru$, $^{188}Re$, $^{186}Re$, and $^{199}Au$.

**16.** The kit of claim 12, further comprising a transfer ligand, a bulking agent, a buffer, a stabilization aid, a solubilization aid or a bacteriostat, in particular, wherein the bulking agent is selected from maltose, sucrose, and hydroxypropyl-γ-cyclodextrin.

**17.** The kit of claim 5, where the oxidant is coenzyme $Q_0$, and the kit further comprises a bulking agent selected from maltose; sucrose, and hydroxypropyl-γ-cyclodextrin.

**18.** The kit of claim 17, wherein the ligand and the coenzyme $Q_0$ are in one vial.

**19.** The kit of claim 17, further comprising a reducing agent, in particular, wherein the reducing agent is selected from stannous chloride, stannous pyrophosphate, stannous fluoride, stannous tartrate, stannous glucoheptonate, stannous DTPA, a borohydride salt, sodium dithionite, a Cu(I) salt, and a formamidine sulphinic acid, in particular, wherein the reducing agent is a stannous compound, in particular, wherein the stannous and the oxidant are present in a ratio from 1:0.39 to 1:25.

**20.** The kit of claim 17, wherein the radiopharmaceutical comprises a radionuclide selected from $^{99m}Tc$, $^{51}Cr$ , $^{67}Cu$, $^{97}Ru$, $^{188}Re$, $^{186}Re$, and $^{199}Au$.

**21.** The kit of claim 17, further comprising a transfer ligand, a bulking agent, a buffer, a stabilization aid, a solubilization aid or a bacteriostat, in particular, wherein the bulking agent is selected from maltose, sucrose, and hydroxypropyl-γ-cyclodextrin.

**22.** A pharmaceutical composition comprising a metal radionuclide-containing radiopharmaceutical and an oxidant, wherein the oxidant has the following structure:

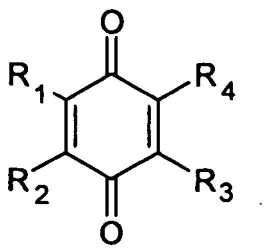

or

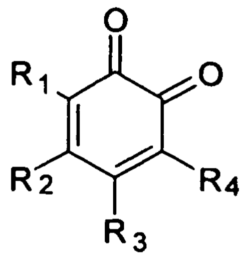

where $R_1$, $R_2$, $R_3$, and $R_4$ are independently selected from hydrogen, hydroxyl, halogen, methyl, alkoxy, aryl, heteroaryl, $NO_2$ and

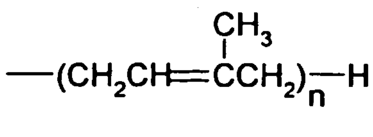

where n is an integer from 0 to 12; or where $R_1$ and $R_2$ taken together form a ring and $R_3$ and $R_4$ are as defined above, and the radiopharmaceutical has a radiochemical purity of greater than 90% at six hours after reconstitution.

**23.** The composition of claim 22, wherein the oxidant is introduced prior to complex formation.

**24.** The composition of claim 22, wherein the oxidant has the following structure:

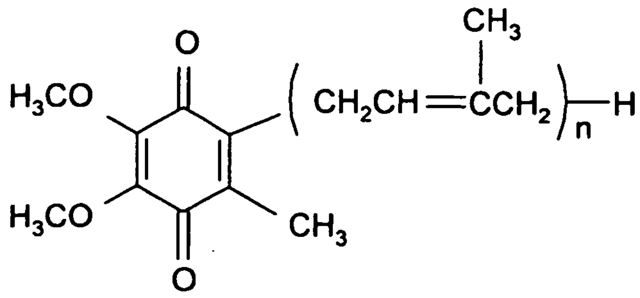

where n is an integer from 0 to 12, in particular, wherein the oxidant is coenzyme $Q_0$, in particular, wherein the radiopharmaceutical comprises a compound of formula I:

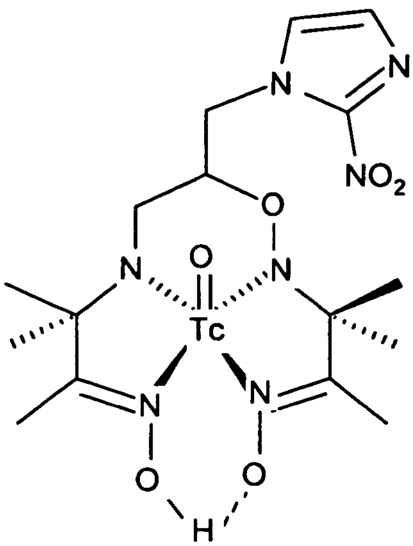

Formula I

**25.** The composition of claim 24, further comprising a transfer ligand, a bulking agent, a buffer, a stabilization aid, a solubilization aid or a bacteriostat, in particular, wherein the bulking agent is selected from maltose, sucrose, and hydroxypropyl-γ-cyclodextrin.

**26.** The composition of claim 22, wherein the radiopharmaceutical comprises a radionuclide selected from $^{99m}Tc$, $^{51}Cr$, $^{67}Cu$, $^{97}Ru$, $^{188}Re$, $^{186}Re$, and $^{199}Au$.

**27.** The composition of claim 22, wherein the composition has a radiochemical purity of greater than 92% at six hours after reconstitution, in particular, wherein the composition has a radiochemical purity of greater than 94% at six hours after reconstitution, in particular, wherein the composition has a radiochemical purity of greater than 96% at six hours after reconstitution.

**28.** A method of making a metal radionuclide-containing radiopharmaceutical composition, comprising the steps of:

a) contacting a complexing ligand with an oxidant wherein the oxidant has the following structure:

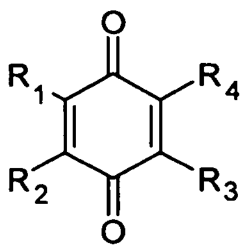

or

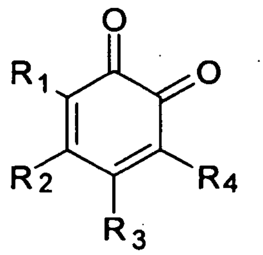

where $R_1$, $R_2$, $R_3$, and $R_4$ are independently selected from hydrogen, hydroxyl, halogen, methyl, alkoxy, aryl, heteroaryl, $NO_2$ and

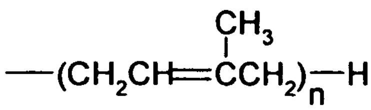

where n is an integer from 0 to 12; or where $R_1$ and $R_2$ taken together form a ring and $R_3$ and $R_4$ are as defined above;

b) followed by contacting the complexing ligand with the radionuclide to form a complex, wherein the radiopharmaceutical composition has a radiochemical purity of greater than 90% at six hours after reconstitution.

**29.** The method of claim 28, further comprising the step of contacting a reducing agent with a radionuclide source to generate the radionuclide, in particular, further comprising the step of reducing a radionuclide source with a transfer ligand to generate the radionuclide.

**30.** The method of claim 28, wherein the oxidant has the following structure:

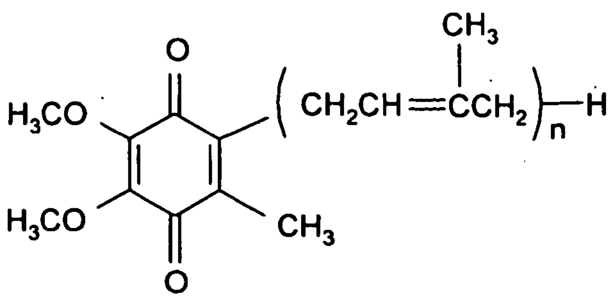

where n is an integer from 0 to 12, in particular, wherein the oxidant is coenzyme $Q_0$.

**31.** The method of claim 28, wherein the complexing ligand comprises a compound of Formula II:

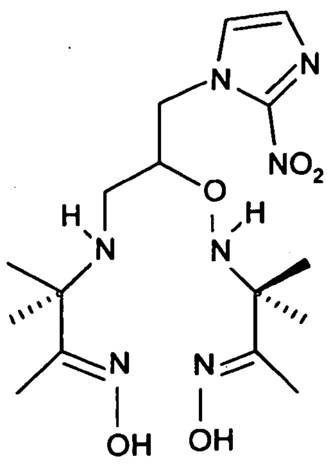

Formula II

in particular,
wherein the radiopharmaceutical comprises a compound of Formula I:

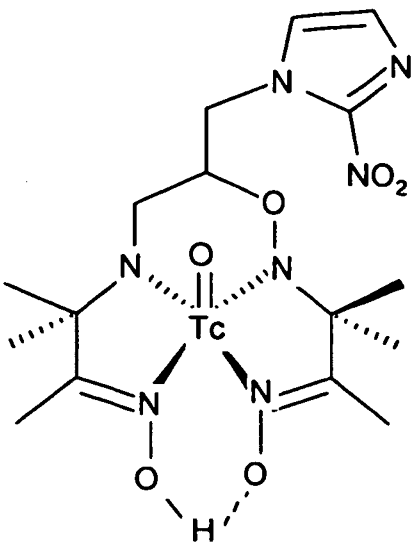

Formula I

**32.** The method of claim 28, wherein the radionuclide is selected from $^{99m}Tc$, $^{51}Cr$, $^{67}Cu$, $^{97}Ru$, $^{188}Re$, $^{186}Re$, and $^{199}Au$.

**33.** The method of claim 28, wherein the radiopharmaceutical composition has a radiochemical purity of greater than 92% approximately six hours after the complex is formed, in particular, wherein the radiopharmaceutical composition has a radiochemical purity of greater than 94% approximately six hours after the complex is formed, in particular, wherein the radiopharmaceutical composition has a radiochemical purity of greater than 96% approximately six hours after the complex is formed.

**34.** A radiopharmaceutical made by the method of claim 28.

**Patentansprüche**

1. Kit zum Herstellen eines ein Metall-Radionuklid enthaltenden Radiopharmazeutikums, wobei das Kit eine vorbestimmte Menge eines komplexierenden Liganden und eine vorbestimmte Menge eines Oxidationsmittels umfaßt, wobei das Oxidationsmittel die folgende Struktur hat:

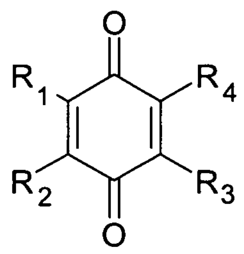

oder

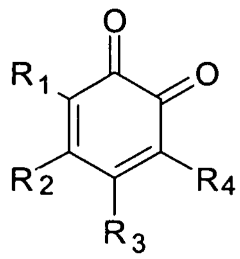

,

wobei $R_1$, $R_2$, $R_3$ und $R_4$ unabhängig ausgewählt sind aus Wasserstoff, Hydroxyl, Halogen, Methyl, Alkoxy, Aryl, Heteroaryl, $NO_2$ und

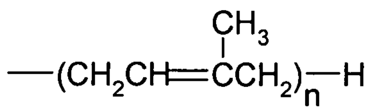

,

wobei n eine ganze Zahl von 0 bis 12 ist; oder wobei $R_1$ und $R_2$ zusammengenommen einen Ring ausbilden und $R_3$ und $R_4$ wie vorstehend definiert sind, und das Radiopharmazeutikum eine radiochemische Reinheit von größer als 90 % sechs Stunden nach Rekonstitution hat, insbesondere, wobei die radiochemische Reinheit größer ist als 92 % sechs Stunden nach Rekonstitution, insbesondere, wobei die radiochemische Reinheit größer ist als 95 % sechs Stunden nach Rekonstitution.

2. Kit nach Anspruch 1, wobei das Oxidationsmittel vor der Komplexbildung eingebracht wird.

3. Kit nach Anspruch 1, wobei das Oxidationsmittel die folgende Struktur hat:

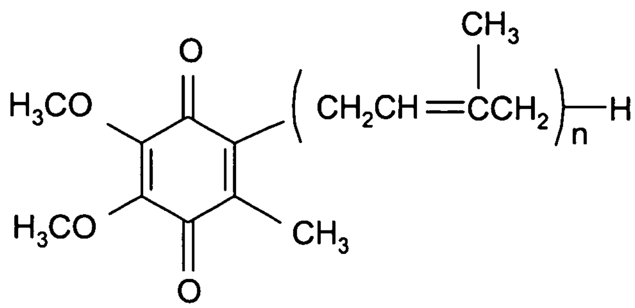

,

wobei n eine ganze Zahl von 0 bis 12 ist, insbesondere, wobei das Oxidationsmittel Coenzym $Q_0$ ist.

4. Kit nach Anspruch 1, wobei der Ligand und das Oxidationsmittel in einem Gefäß sind.

5. Kit nach Anspruch 1, wobei der komplexierende Ligand eine Verbindung der Formel II umfasst:

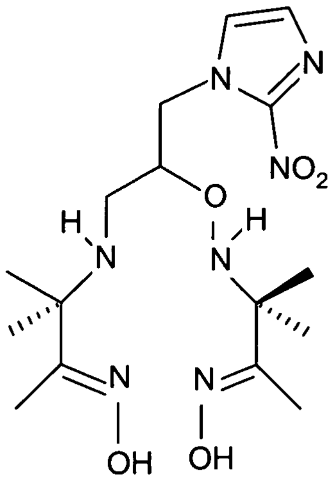

Formel II

6. Kit nach Anspruch 1, ferner umfassend ein Reduktionsmittel, insbesondere, wobei das Reduktionsmittel ausgewählt ist aus Zinnchlorid, Zinnpyrophosphat, Zinnfluorid, Zinntartrat, Zinnglucoheptonat, Zinn-DTPA, einem Borhydridsalz, Natriumdithionit, einem Cu(I)-Salz und einer Formamidin-Sulfinsäure, insbesondere, wobei das Reduktionsmittel eine Zinnverbindung ist, insbesondere, wobei das Zinn und das Oxidationsmittel in einem Verhältnis von 1:0,39 bis 1:25 anwesend sind.

7. Kit nach Anspruch 1, wobei das Radiopharmazeutikum ein Radionuklid, ausgewählt aus $^{99m}$Tc, $^{51}$Cr, $^{67}$Cu, $^{97}$Ru, $^{188}$Re, $^{186}$Re und $^{199}$Au, umfasst.

8. Kit nach Anspruch 1, ferner umfassend einen Transferliganden, einen Füllstoff (bulking agent), einen Puffer, eine Stabilisierungshilfe, einen Lösungsvermittler (solubilization aid) oder ein Bakteriostat, insbesondere, wobei der Füllstoff ausgewählt ist aus Maltose, Saccharose und Hydroxypropyl-$\gamma$-cyclodextrin.

9. Kit für die Herstellung eines ein Metall-Radionuklid enthaltenden Radiopharmazeutikums, wobei das Kit eine lyophilisierte Zusammensetzung umfaßt, die eine vorbestimmte Menge eines komplexierenden Liganden und eine vorbestimmte Menge eines Oxidationsmittels umfaßt, wobei das Oxidationsmittel die folgende Struktur hat:

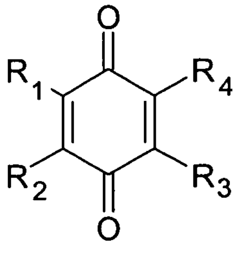

oder

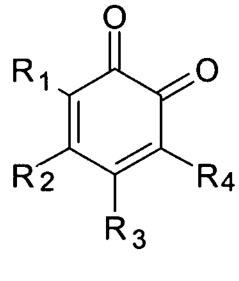

,

wobei $R_1$, $R_2$, $R_3$ und $R_4$ unabhängig ausgewählt sind aus Wasserstoff, Hydroxyl, Halogen, Methyl, Alkoxy, Aryl, Heteroaryl, $NO_2$ und

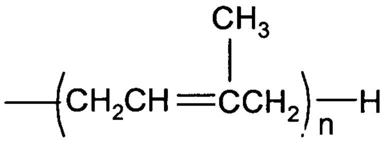

,

wobei n eine ganze Zahl von 0 bis 12 ist; oder wobei $R_1$ und $R_2$ zusammengenommen einen Ring ausbilden und $R_3$ und $R_4$ wie vorstehend definiert sind.

**10.** Kit nach Anspruch 9, wobei das Radiopharmazeutikum eine radiochemische Reinheit von größer als 90 % sechs Stunden nach Rekonstitution hat, insbesondere wobei die radiochemische Reinheit größer als 95 % sechs Stunden nach Rekonstitution ist.

**11.** Kit nach Anspruch 9, wobei der Ligand und das Oxidationsmittel in einem Gefäß sind.

**12.** Kit nach Anspruch 9, wobei das Oxidationsmittel die folgende Struktur hat:

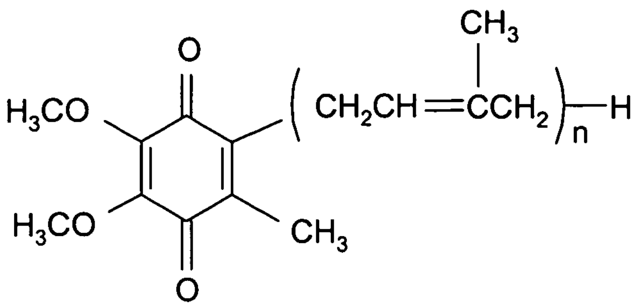

,

wobei n eine ganze Zahl von 0 bis 12 ist, insbesondere, wobei das Oxidationsmittel Coenzym $Q_0$ ist.

**13.** Kit nach Anspruch 12, wobei die radiochemische Reinheit größer als 92 % sechs Stunden nach Rekonstitution ist.

**14.** Kit nach Anspruch 12, ferner umfassend ein Reduktionsmittel, insbesondere, wobei das Reduktionsmittel ausgewählt ist aus Zinnchlorid, Zinnpyrophosphat, Zinnfluorid, Zinntartrat, Zinnglucoheptonat, Zinn-DTPA, einem Borhydridsalz, Natriumdithionit, einem Cu(I)-Salz und einer Formamidin-Sulfinsäure, insbesondere, wobei das Reduktionsmittel eine Zinnverbindung ist, insbesondere, wobei das Zinn und das Oxidationsmittel in einem Verhältnis von 1:0,39 bis 1:25 anwesend sind.

**15.** Kit nach Anspruch 12, wobei das Radiopharmazeutikum ein Radionuklid, ausgewählt aus $^{99m}$Tc, $^{51}$Cr, $^{67}$Cu, $^{97}$Ru, $^{188}$Re, $^{186}$Re und $^{199}$Au, umfaßt.

**16.** Kit nach Anspruch 12, ferner umfassend einen Transferliganden, einen Füllstoff (bulking agent), einen Puffer, eine Stabilisierungshilfe, einen Lösungsvermittler (solubilization aid) oder ein Bakteriostat, insbesondere, wobei der Füllstoff ausgewählt ist aus Maltose, Saccharose und Hydroxypropyl-$\gamma$-cyclodextrin.

**17.** Kit nach Anspruch 5, wobei das Oxidationsmittel Coenzym $Q_0$ ist, und das Kit ferner einen Füllstoff (bulking agent) umfaßt, ausgewählt aus Maltose, Saccharose und Hydroxypropyl-$\gamma$-cyclodextrin.

**18.** Kit nach Anspruch 17, wobei der Ligand und das Coenzym $Q_0$ in einem Gefäß sind.

**19.** Kit nach Anspruch 17, ferner umfassend ein Reduktionsmittel, insbesondere, wobei das Reduktionsmittel ausgewählt ist aus Zinnchlorid, Zinnpyrophosphat, Zinnfluorid, Zinntartrat, Zinnglucoheptonat, Zinn-DTPA, einem Borhydridsalz, Natriumdithionit, einem Cu(I)-Salz und einer Formamidin-Sulfinsäure ist, insbesondere, wobei das Reduktionsmittel

eine Zinn-Verbindung ist, insbesondere, wobei das Zinn und das Oxidationsmittel in einem Verhältnis von 1:0,39 bis 1:25 anwesend sind.

**20.** Kit nach Anspruch 17, wobei das Radiopharmazeutikum ein Radionuklid, ausgewählt aus $^{99m}Tc$, $^{51}Cr$, $^{67}Cu$, $^{97}Ru$, $^{188}Re$, $^{186}Re$ und $^{199}Au$, umfaßt.

**21.** Kit nach Anspruch 17, ferner umfassend einen Transferliganden, einen Füllstoff (bulking agent), einen Puffer, eine Stabilisierungshilfe, einen Lösungsvermittler (subilization aid) oder ein Bakteriostat, insbesondere, wobei der Füllstoff ausgewählt ist aus Maltose, Saccharose und Hydroxypropyl-γ-cyclodextrin.

**22.** Pharmazeutische Zusammensetzung, umfassend ein Metall-Radionuklid enthaltendes Radiopharmazeutikum und ein Oxidationsmittel, wobei das Oxidationsmittel die folgende Struktur hat:

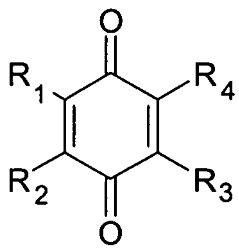

oder

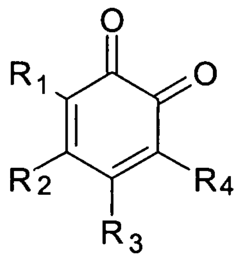

,

wobei $R_1$, $R_2$, $R_3$ und $R_4$ unabhängig ausgewählt sind aus Wasserstoff, Hydroxyl, Halogen, Methyl, Alkoxy, Aryl, Heteroaryl, $NO_2$ und

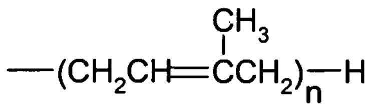

,

wobei n eine ganze Zahl von 0 bis 12 ist; oder wobei $R_1$ und $R_2$ zusammengenommen einen Ring ausbilden und $R_3$ und $R_4$ wie vorstehend definiert sind, und das Radiopharmazeutikum eine radiochemische Reinheit von größer als 90 % sechs Stunden nach Rekonstitution hat.

**23.** Zusammensetzung nach Anspruch 22, wobei das Oxidationsmittel vor der Komplexbildung eingebracht wird.

**24.** Zusammensetzung nach Anspruch 22, wobei das Oxidationsmittel die folgende Struktur hat:

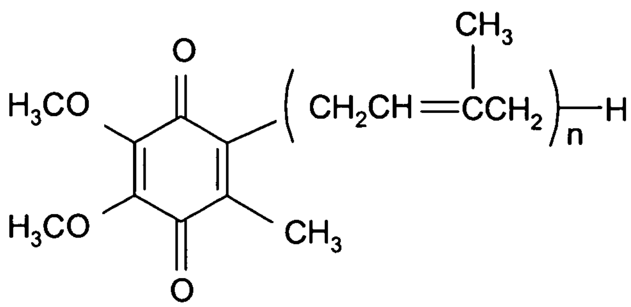

,

wobei n eine ganze Zahl von 0 bis 12 ist, insbesondere, wobei das Oxidationsmittel Coenzym $Q_0$ ist, insbesondere, wobei das Radiopharmazeutikum eine Verbindung der Formel I umfaßt:

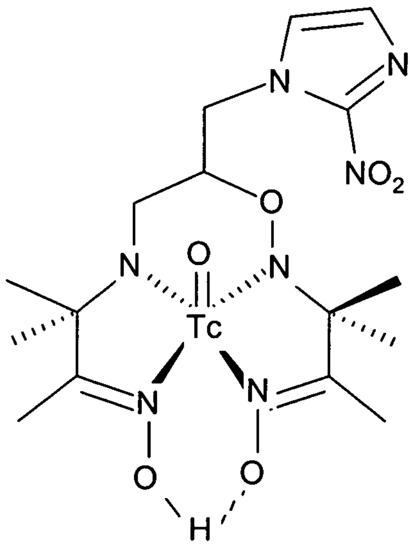

Formel I

**25.** Zusammensetzung nach Anspruch 24, ferner umfassend einen Transferliganden, einen Füllstoff (bulking agent), einen Puffer, eine Stabilisierungshilfe, einen Lösungsvermittler (solubilization aid) oder ein Bakteriostat, insbesondere, wobei der Füllstoff ausgewählt ist aus Maltose, Saccharose und Hydroxypropyl-γ-cyclodextrin.

**26.** Zusammensetzung nach Anspruch 22, wobei das Radiopharmazeutikum ein Radionuklid, ausgewählt aus $^{99m}Tc$, $^{51}Cr$, $^{67}Cu$, $^{97}Ru$, $^{188}Re$, $^{186}Re$ und $^{199}Au$, umfaßt.

**27.** Zusammensetzung nach Anspruch 22, wobei die Zusammensetzung eine radiochemische Reinheit von größer als 92 % sechs Stunden nach Rekonstitution hat, insbesondere, wobei die Zusammensetzung eine radiochemische Reinheit von größer als 94 % sechs Stunden nach Rekonstitution hat, insbesondere, wobei die Zusammensetzung eine radiochemische Reinheit von größer als 96 % sechs Stunden nach Rekonstitution hat.

**28.** Verfahren zum Herstellen einer ein Metall-Radionuklid enthaltenden radiopharmazeutischen Zusammensetzung, umfassend die Schritte:

a) Inkontaktbringen eines komplexierenden Liganden mit einem Oxidationsmittel, wobei das Oxidationsmittel die folgende Struktur hat:

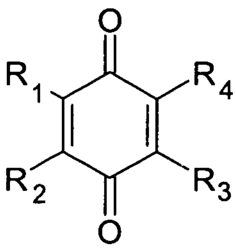

oder

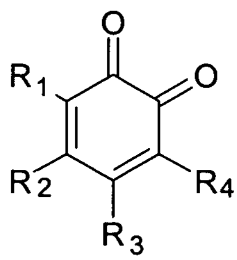

,

wobei $R_1$, $R_2$, $R_3$ und $R_4$ unabhängig ausgewählt sind aus Wasserstoff, Hydroxyl, Halogen, Methyl, Alkoxy, Aryl, Heteroaryl, $NO_2$ und

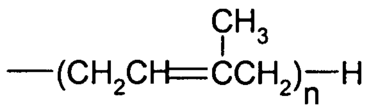

,

wobei n eine ganze Zahl von 0 bis 12 ist; oder wobei $R_1$ und $R_2$ zusammengenommen einen Ring ausbilden und $R_3$ und $R_4$ wie vorstehend definiert sind;

b) gefolgt von Inkontaktbringen des komplexierenden Liganden mit dem Radionuklid, um einen Komplex auszubilden, wobei die radiopharmazeutische Zusammensetzung eine radiochemische Reinheit von größer als 90 % sechs Stunden nach Rekonstitution hat.

**29.** Verfahren nach Anspruch 28, ferner umfassend den Schritt des Inkontaktbringens eines Reduktionsmittels mit einer Radionuklid-Quelle, um das Radionuklid zu generieren, insbesondere, ferner umfassend den Schritt des Reduzierens einer Radionuklid-Quelle mit einem Transferliganden, um das Radionuklid zu generieren.

**30.** Verfahren nach Anspruch 28, wobei das Oxidationsmittel die folgende Struktur hat:

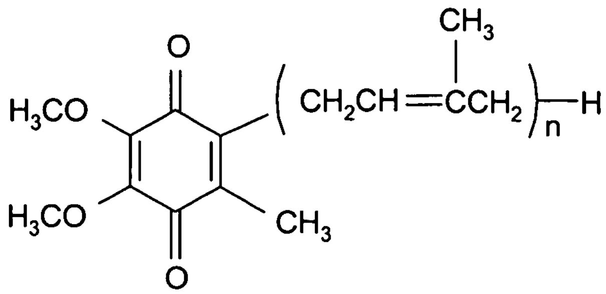

,

wobei n eine ganze Zahl von 0 bis 12 ist, insbesondere, wobei das Oxidationsmittel Coenzym $Q_0$ ist.

**31.** Verfahren nach Anspruch 28, wobei der komplexierende Ligand eine Verbindung der Formel II umfasst:

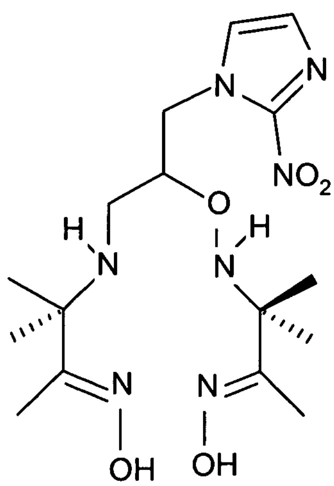

Formel II

insbesondere,
wobei das Radiopharmazeutikum eine Verbindung der Formel I umfasst:

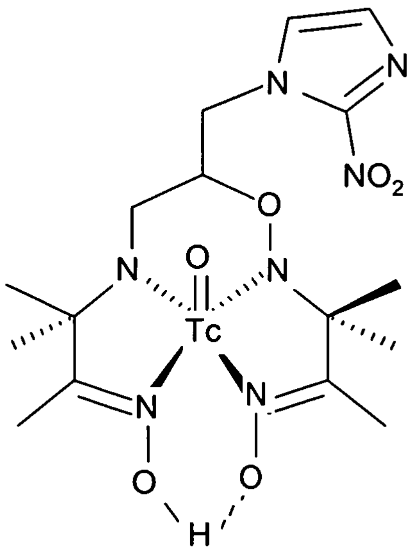

Formel I

**32.** Verfahren nach Anspruch 28, wobei das Radionuklid ausgewählt ist aus $^{99m}Tc$, $^{67}Cu$, $^{97}Ru$, $^{188}Re$, $^{186}Re$ und $^{199}Au$.

**33.** Verfahren nach Anspruch 28, wobei die radiopharmazeutische Zusammensetzung eine radiochemische Reinheit von größer als 92 % hat ungefähr sechs Stunden nachdem der Komplex gebildet wird, insbesondere wobei die radiopharmazeutische Zusammensetzung eine radiochemische Reinheit von größer als 94 % hat ungefähr sechs Stunden nachdem der Komplex gebildet wird, insbesondere wobei die radiopharmazeutische Zusammensetzung eine radiochemische Reinheit von größer als 96 % hat ungefähr sechs Stunden nachdem der Komplex gebildet wird.

**34.** Radiopharmazeutikum, hergestellt durch das Verfahren nach Anspruch 28.

**Revendications**

**1.** Matériel pour préparer un produit radiopharmaceutique contenant un radionucléide métallique, le matériel comprenant une quantité prédéterminée d'un ligand de complexation et une quantité prédéterminée d'un oxydant, où l'oxydant a la structure suivante :

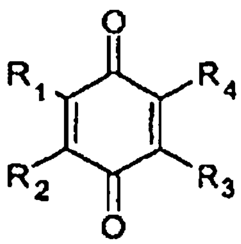

ou

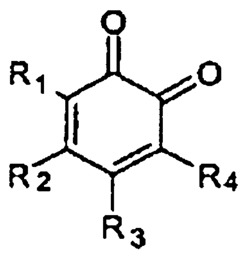

où $R_1$, $R_2$, $R_3$ et $R_4$ sont indépendamment choisis parmi un atome d'hydrogène, un groupe hydroxyle, un atome d'halogène, un groupe méthyle, alcoxy, aryle, hétéroaryle, $NO_2$ et

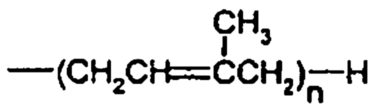

où n est un nombre entier de 0 à 12 ; ou bien où $R_1$ et $R_2$ pris ensemble forment un cycle et $R_3$ et $R_4$ sont tels que définis ci-dessus, et le produit radiopharmaceutique a une pureté radiochimique de plus de 90 % à six heures après reconstitution, en particulier dans lequel la pureté radiochimique est supérieure à 92 % à six heures après reconstitution, en particulier dans lequel la pureté radiochimique est supérieure à 95 % à six heures après reconstitution.

**2.** Matériel selon la revendication 1, dans lequel l'oxydant est introduit préalablement à la formation de complexe.

**3.** Matériel selon la revendication 1, dans lequel l'oxydant a la structure suivante :

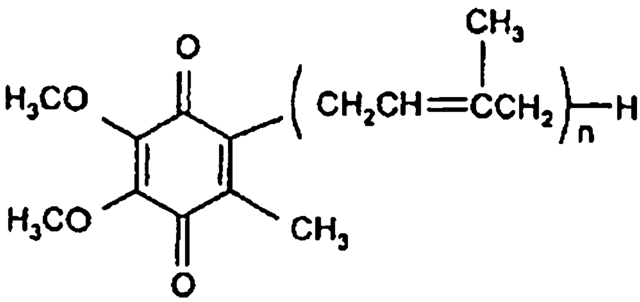

où n est un nombre entier de 0 à 12, en particulier où l'oxydant est la coenzyme $Q_0$.

**4.** Matériel selon la revendication 1, dans lequel le ligand et l'oxydant sont dans un flacon.

**5.** Matériel selon la revendication 1, dans lequel le ligand de complexation comprend un composé de formule II :

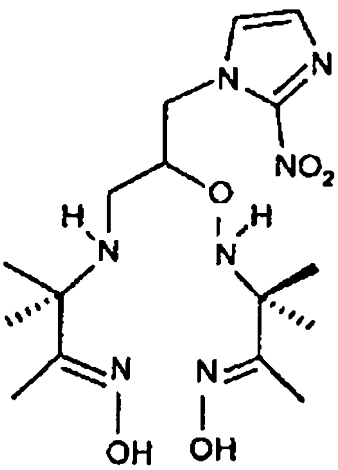

Formule II

**6.** Matériel selon la revendication 1, comprenant en outre un agent réducteur, en particulier où l'agent réducteur est choisi parmi le chlorure stanneux, le pyrophosphate stanneux, le fluorure stanneux, le tartrate stanneux, le glucoheptonate stanneux, le DTPA stanneux, un sel de borohydrure, le dithionite de sodium, un sel de Cu(I) et un acide formamidine sulfinique, en particulier où l'agent réducteur est un composé stanneux, en particulier où le composé stanneux et l'oxydant sont présents en un rapport de 1 : 0,39 à 1 : 25.

**7.** Matériel selon la revendication 1, dans lequel le produit radiopharmaceutique comprend un radionucléide choisi parmi $^{99m}Tc$, $^{51}Cr$, $^{67}Cu$, $^{97}Ru$, $^{188}Re$, $^{186}Re$ et $^{199}Au$.

**8.** Matériel selon la revendication 1, comprenant en outre un ligand de transfert, un agent diluant, un tampon, une aide à la stabilisation, une aide à la solubilisation ou un bactériostat, en particulier où l'agent diluant est choisi parmi le maltose, le saccharose et l'hydroxypropyl-γ-cyclodextrine.

**9.** Matériel pour la préparation d'un produit radiopharmaceutique contenant un radionucléide métallique, le matériel comprenant une composition lyophilisée comprenant une quantité prédéterminée d'un ligand de complexation et une quantité prédéterminée d'un oxydant, où l'oxydant a la structure suivante :

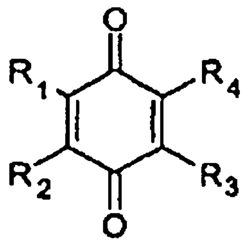

ou

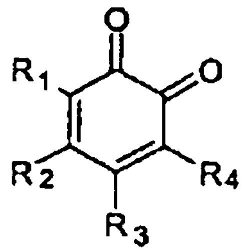

où $R_1$, $R_2$, $R_3$ et $R_4$ sont indépendamment choisis parmi un atome d'hydrogène, un groupe hydroxyle, un atome d'halogène, un groupe méthyle, alcoxy, aryle, hétéroaryle, $NO_2$ et

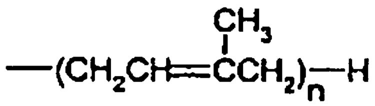

où n est un nombre entier de 0 à 12 ; ou bien où $R_1$ et $R_2$ pris ensemble forment un cycle et $R_3$ et $R_4$ sont tels que définis ci-dessus.

**10.** Matériel selon la revendication 9, dans lequel le produit radiopharmaceutique a une pureté radiochimique supérieure à 90 % à six heures après reconstitution, en particulier où la pureté radiochimique est supérieure à 95 % à six heures après reconstitution.

**11.** Matériel selon la revendication 9, dans lequel le ligand et l'oxydant sont dans un flacon.

**12.** Matériel selon la revendication 9, dans lequel l'oxydant a la structure suivante :

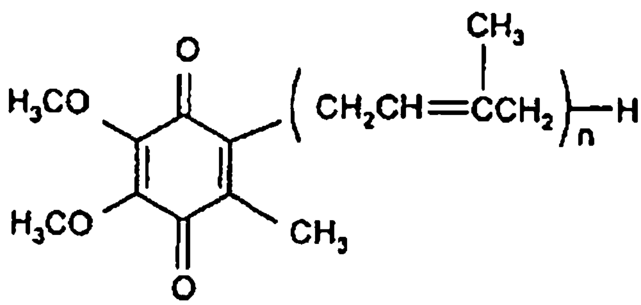

où n est un nombre entier de 0 à 12, en particulier où l'oxydant est la coenzyme $Q_0$.

**13.** Matériel selon la revendication 12, dans lequel la pureté radiochimique est supérieure à 92 % à six heures après

reconstitution.

**14.** Matériel selon la revendication 12, comprenant en outre un agent réducteur, en particulier où l'agent réducteur est choisi parmi le chlorure stanneux, le pyrophosphate stanneux, le fluorure stanneux, le tartrate stanneux, le glucoheptonate stanneux, le DTPA stanneux, un sel de borohydrure, le dithionite de sodium, un sel de Cu(I) et un acide formamidine sulfinique, en particulier où l'agent réducteur est un composé stanneux, en particulier où le composé stanneux et l'oxydant sont présents en un rapport de 1 : 0,39 à 1 : 25.

**15.** Matériel selon la revendication 12, dans lequel le produit radiopharmaceutique comprend un radionucléide choisi parmi $^{99m}Tc$, $^{51}Cr$, $^{67}Cu$, $^{97}Ru$, $^{188}Re$, $^{186}Re$ et $^{199}Au$.

**16.** Matériel selon la revendication 12, comprenant en outre un ligand de transfert, un agent diluant, un tampon, un aide à la stabilisation, une aide à la solubilisation ou un bactériostat, en particulier où l'agent diluant est choisi parmi le maltose, le saccharose et l'hydroxypropyl-γ-cyclodextrine.

**17.** Matériel selon la revendication 5, dans lequel l'oxydant est la coenzyme $Q_0$, et le matériel comprend en outre un agent diluant choisi parmi le maltose, le saccharose et l'hydroxypropyl-γ-cyclodextrine.

**18.** Matériel selon la revendication 17, dans lequel le ligand et la coenzyme $Q_0$ sont dans un flacon.

**19.** Matériel selon la revendication 17, comprenant en outre un agent réducteur en particulier où l'agent réducteur est choisi parmi le chlorure stanneux, le pyrophosphate stanneux, le fluorure stanneux, le tartrate stanneux, le glucoheptonate stanneux, le DTPA stanneux, un sel de borohydrure, le dithionite de sodium, un sel de Cu(I) et un acide formamidine sulfinique, en particulier où l'agent réducteur est un composé stanneux, en particulier où le composé stanneux et l'oxydant sont présents en un rapport de 1 : 0,39 à 1 : 25.

**20.** Matériel selon la revendication 17, dans lequel le produit radiopharmaceutique comprend un radionucléide choisi parmi $^{99m}Tc$, $^{51}Cr$, $^{67}Cu$, $^{97}Ru$, $^{188}Re$, $^{186}Re$ et $^{199}Au$.

**21.** Matériel selon la revendication 17, comprenant en outre un ligand de transfert, un agent diluant, un tampon, une aide à la stabilisation, une aide à la solubilisation ou un bactériostat, en particulier où l'agent diluant est choisi parmi le maltose, le saccharose et l'hydroxypropyl-γ-cyclodextrine.

**22.** Composition pharmaceutique comprenant un produit radiopharmaceutique contenant un radionucléide métallique et un oxydant, où l'oxydant a la structure suivante :

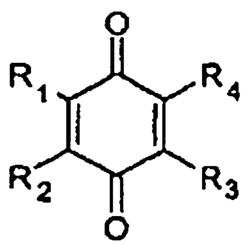

ou

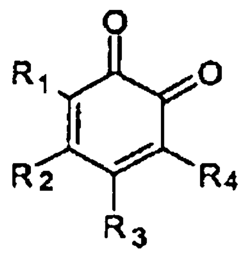

où $R_1$, $R_2$, $R_3$ et $R_4$ sont indépendamment choisis parmi un atome d'hydrogène, un groupe hydroxyle, un atome d'halogène, un groupe méthyle, alcoxy, aryle, hétéroaryle, $NO_2$ et

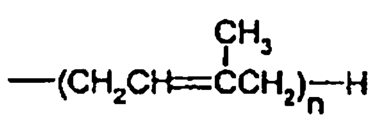

où n est un nombre entier de 0 à 12 ; ou bien où $R_1$ et $R_2$ pris ensemble forment un cycle et $R_3$ et $R_4$ sont tels que définis ci-dessus, et le produit radiopharmaceutique a une pureté radiochimique de plus de 90 % à six heures après

reconstitution.

**23.** Composition selon la revendication 22, dans laquelle l'oxydant est introduit préalablement à la formation de complexe.

**24.** Composition selon la revendication 22, dans laquelle l'oxydant a la structure suivante :

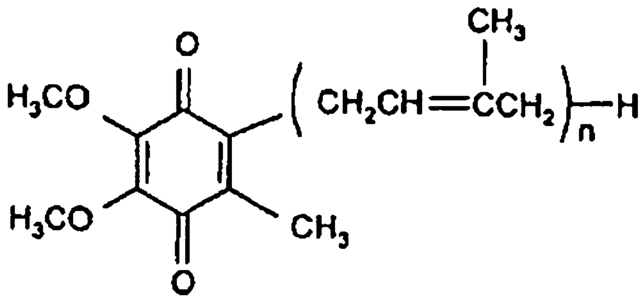

où n est un nombre entier de 0 à 12, en particulier où l'oxydant est la coenzyme $Q_0$, en particulier où le produit radiopharmaceutique comprend un composé de formule 1 :

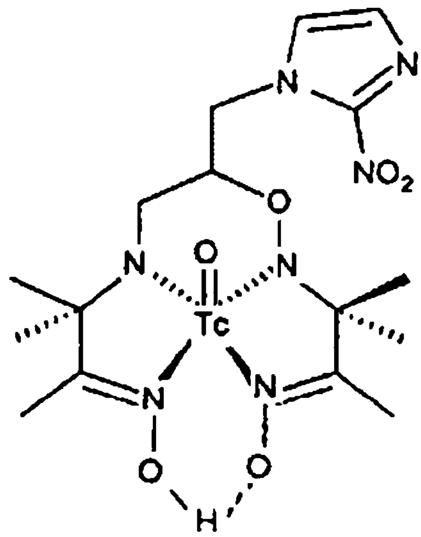

Formule I

**25.** Composition selon la revendication 24, comprenant en outre un ligand de transfert, un agent diluant, un tampon, une aide à la stabilisation, une aide à la solubilisation ou un bactériostat, en particulier où l'agent diluant est choisi parmi le maltose, le saccharose et l'hydroxypropyl-γ-cyclodextrine.

**26.** Composition selon la revendication 22, dans laquelle le produit radiopharmaceutique comprend un radionucléide choisi parmi $^{99m}Tc$, $^{51}Cr$, $^{67}Cu$, $^{97}Ru$, $^{188}Re$, $^{186}Re$ et $^{199}Au$.

**27.** Composition selon la revendication 22, dans laquelle la composition a une pureté radiochimique supérieure à 92 % à six heures après reconstitution, en particulier dans laquelle la composition a une pureté radiochimique supérieure à 94 % à six heures après reconstitution, en particulier dans laquelle la composition a une pureté radiochimique supérieure à 96 % à six heures après reconstitution.

**28.** Procédé de préparation d'une composition radiopharmaceutique contenant un radionucléide métallique, comprenant les étapes consistant à :

a) mettre en contact un ligand de complexation avec un oxydant, où l'oxydant a la structure suivante :

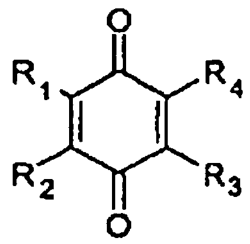

ou

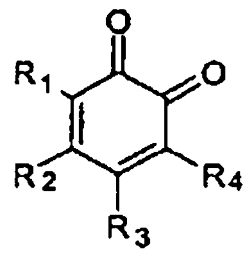

où $R_1$, $R_2$, $R_3$ et $R_4$ sont indépendamment choisis parmi un atome d'hydrogène, un groupe hydroxyle, un atome d'halogène, un groupe méthyle, alcoxy, aryle, hétéroaryle, $NO_2$ et

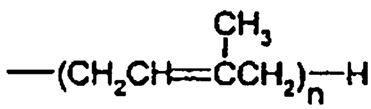

où n est un nombre entier de 0 à 12 ; ou bien où $R_1$ et $R_2$ pris ensemble forment un cycle et $R_3$ et $R_4$ sont tels que définis ci-dessus ;

b) mettre ensuite en contact le ligand de complexation avec le radionucléide pour former un complexe, où la composition radiopharmaceutique a une pureté radiochimique de plus de 90 % à six heures après reconstitution.

**29.** Procédé selon la revendication 28, comprenant en outre l'étape de mise en contact d'un agent réducteur avec une source de radionucléide pour générer le radionucléide, en particulier comprenant en outre l'étape de réduction d'une source de radionucléide avec un ligand de transfert pour générer le radionucléide.

**30.** Procédé selon la revendication 28, dans lequel l'oxydant a la structure suivante :

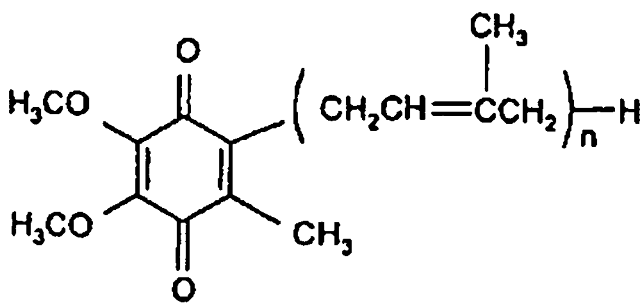

où n est un nombre entier de 0 à 12, en particulier où l'oxydant est la coenzyme $Q_0$.

**31.** Procédé selon la revendication 28, dans lequel le ligand de complexation comprend un composé de formule II :

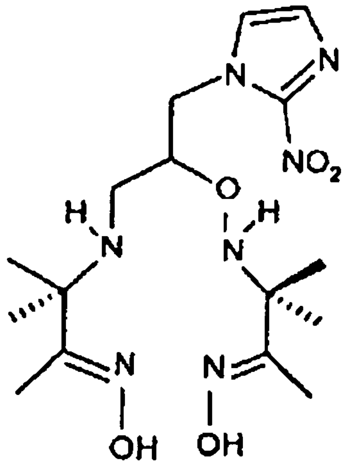

Formule II

en particulier,
où le produit radiopharmaceutique comprend un composé de formule I :

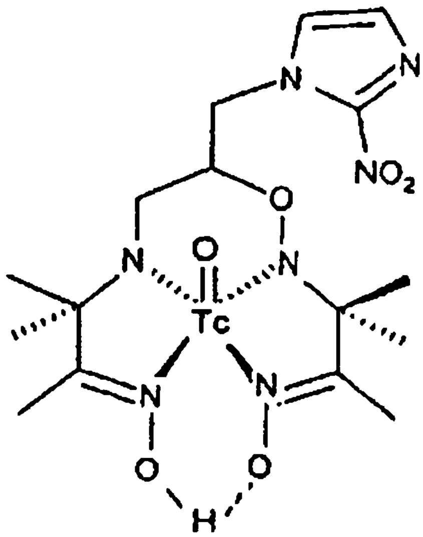

Formule I

**32.** Procédé selon la revendication 28, dans lequel le radionucléide est choisi parmi $^{99m}Tc$, $^{51}Cr$, $^{67}Cu$, $^{97}Ru$, $^{188}Re$, $^{86}Re$ et $^{99}Au$.

**33.** Procédé selon la revendication 28, dans lequel la composition radiopharmaceutique a un pureté radiochimique supérieure à 92 % approximativement six heures après la formation du complexe, en particulier dans lequel la composition radiopharmaceutique a une pureté radiochimique supérieure à 94 % approximativement six heures après la formation du complexe, en particulier dans lequel la composition radiopharmaceutique a une pureté radiochimique supérieure à 96 % approximativement six heures après la formation du complexe.

**34.** Produit radiopharmaceutique préparé par le procédé de la revendication 28.

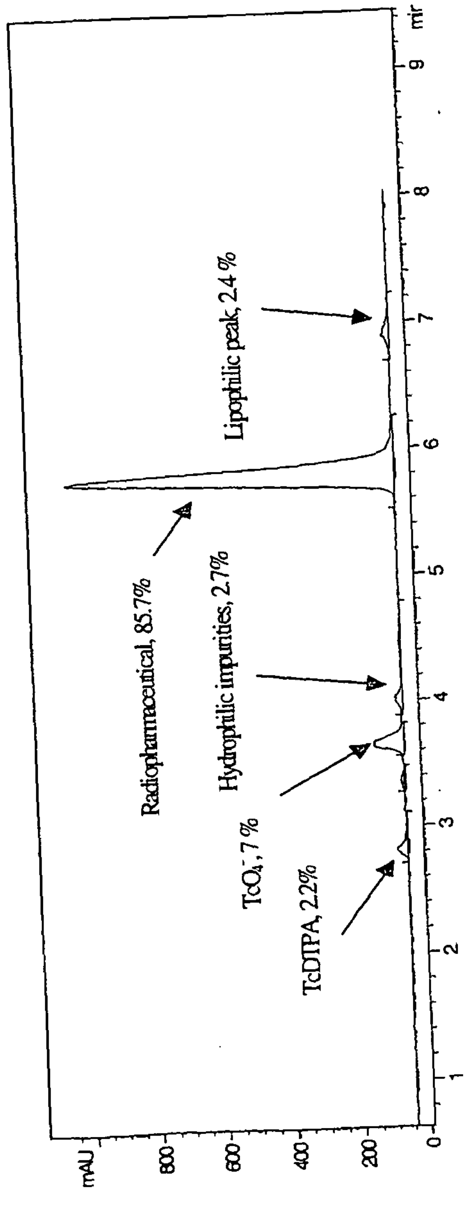
Lipophilic peak, 2.4 %
Radiopharmaceutical, 85.7%
Hydrophilic impurities, 2.7%
$TcO_4^-$, 7 %
TcDTPA, 2.2%
mAU
800
600
400
200
0
1
2
3
4
5
6
7
8
9
min

FIG. 1

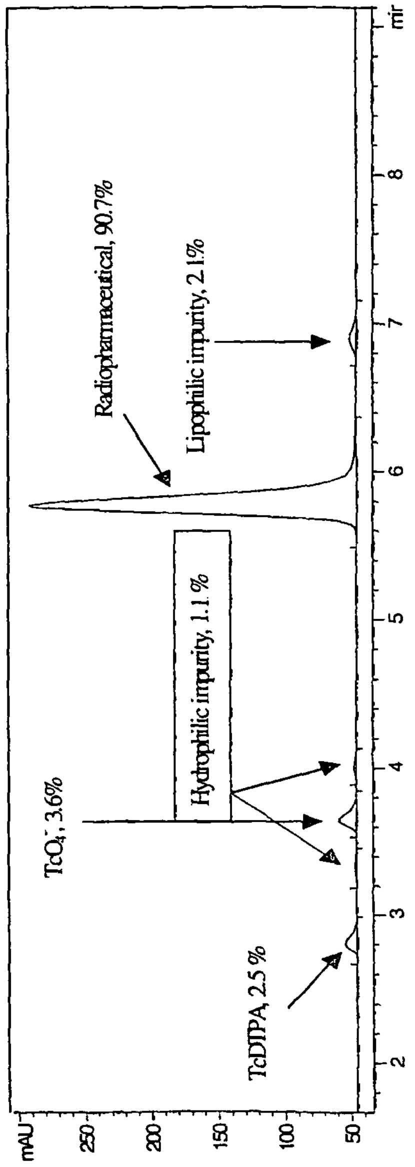
mAU
250
200
150
100
50
2
3
4
5
6
7
8
min
TcDTPA, 2.5 %
$TcO_4^-$, 3.6%
Hydrophilic impurity, 1.1 %
Radiopharmaceutical, 90.7%
Lipophilic impurity, 2.1%

FIG. 2

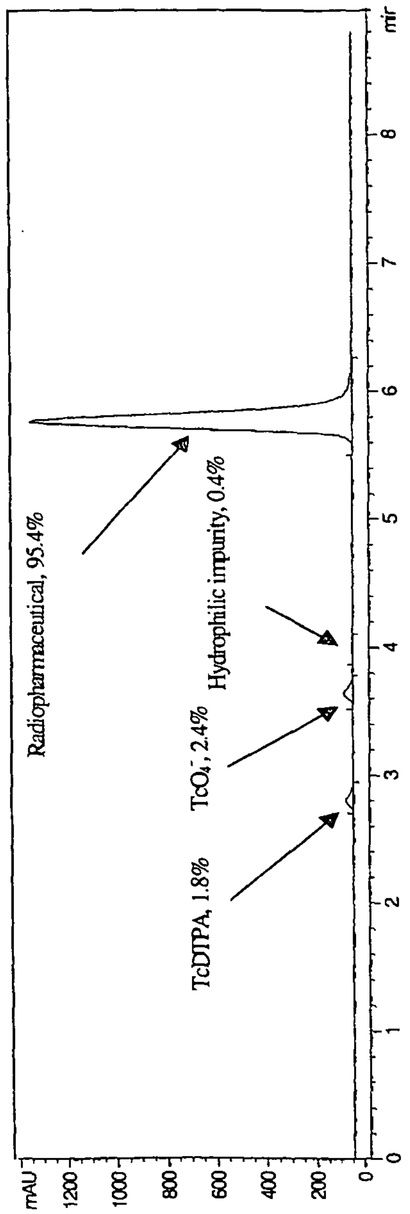
Radiopharmaceutical, 95.4%
Hydrophilic impurity, 0.4%
$TcO_4^-$, 2.4%
TcDTPA, 1.8%
mAU
1200
1000
800
600
400
200
0
0
1
2
3
4
5
6
7
8
min

FIG. 3

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 02092005 A
- GB 2231267 A
- US 5262175 A
- EP 46067 A
- EP 313712 A
- DE 3323375
- US 6093382 A
- US 5608110 A
- US 5665329 A
- US 565654 A
- US 5688487 A
- CA 9400395 W
- CA 9400479 W
- US 5183653 A
- US 5387409 A
- US 5118797 A
- WO 0043004 A


### Non-patent literature cited in the description


- **M. G. Kulkarni et al.** *Tetrahedron Letters,* 1990, vol. 30 (31), 4497-4500
- **Tofe.** *Radiopharm. 2, Proc. Int. Symp., 2nd,* 1979
- **Solanki et al.** *C. B. Nucl. Med. Commun.,* 1994, vol. 15 (9), 718-22
- **Cleyhens et al.** *Lab. Nucl. Med. Radiophann., Nuklearmedizin,* 1991, vol. 27, 133-5
- *Res. Discl.,* 1989, 305-666
- **Liu ; Edwards.** *Chem Rev.,* 1999, vol. 99, 2235-2268
- Imaging of Hypoxia. **H.J. Machulla.** Developments in Nuclear Medicine. Kluver Publ, 1999, vol. 33
- **Thakur et al.** *J. Labelled Compounds and Radiopharmaceuticals,* 1993, vol. 32, 365-367
- **Su, Zi-Fen et al.** *Bioconjugate Chem.,* 2000, vol. 11 (5), 652-663
- **Zhang. Xiuguo et al.** *Bioconjugate Chem.,* 2000, vol. 11 (3), 401-407
- **Edwards DI.** Nitroimidazoles drugs-action and resistance mechanism. I. Mechanism of action. *J. Antimicrob. Chemother.,* 1993, vol. 31, 9-20
- **Hu, Miao Lin ; Chen Ling-Chun ; Sano, Mitsuaki.** Para-aminobenzoic acid scavenges reactive oxygen species and protects DNA against UV and free radical damage. *J. Nutr. Biochem.,* 1995, vol. 6 (9), 504-8